# EUROPEAN PATENT SPECIFICATION

(11) **EP 0 340 109 B1**
(45) Date of publication and mention of the grant of the patent: **28.05.1997**
(21) Application number: 89401190.7
(22) Date of filing: 26.04.1989
(51) Int. Cl.: C12Q 1/68, A61K 39/395, G01N 33/564

(54) **Anti-T-cell receptor determinants as autoimmune disease treatment**
Anti-T-Zell Rezeptordeterminanten zur Behandlung von Autoimmunkrankheiten
Anti- Déterminants du récepteur de la céllule T pour le traitement des maladies autoimmunes

(30) Priority: 28.04.1988 US 188297
(43) Date of publication of application: 02.11.1989
(73) Proprietor: THE BOARD OF TRUSTEES OF THE LELAND STANFORD JUNIOR UNIVERSITY, Stanford California 94305 (US)
(72) Inventor: Steinman, Lawrence, Palo Alto California 94301 (US); Zamvil, Scott S., Belmont California 94002 (US); Mitchell, Dennis J., Morgan Hill California 95037 (US)
(74) Representative: Silveston, Judith

(56) References cited:
- EP-A- 0 097 518
- WO-A-86/06413
- WO-A-87/03600
- CLINICAL RESEARCH, vol. 35, no. 3, 1987, Thorofare (US); R. GOLDSTEIN et al., p. 388A
- JOURNAL OF EXPERIMENTAL MEDICINE, vol. 164, September 1986, New York (US); N. KIMURA et al., pp. 739-750
- MEDLINE, accession no. 88270505; H. ACHA-ORBEA et al.,

## Description

The subject invention concerns diagnosis and treatment of autoimmune diseases.

Autoimmune diseases are a result of a failure of the immune system to avoid recognition of self. The attack by the immune system of host cells can result in a large number of disorders, including such neural diseases as multiple sclerosis and myasthenia gravis, diseases of the joints, such as rheumatoid arthritis, attacks on nucleic acids, as observed with systemic lupus erythematosus and such other diseases associated with various organs, as psoriasis, juvenile onset diabetes, Sjögren's disease, thyroid disease. These diseases can have a variety of symptoms, which can vary from minor and irritating to life-threatening.

In the past decade, there has been an increasing effort to understand the various processes of the immune system and how the immune system may be modulated to enhance the natural system to protect the host against pathogens. The complexity of the immune system has been a daunting barrier to this elucidation. The many different types of cells, their different roles, the nature of their interactions, the molecules involved with their interactions, and the manner in which the various molecules are activated to fulfill their roles, all contribute to the complexity and difficulties in designing experimental procedures. In attempting to understand the mechanisms involved with the immunological response, their is substantial interest in understanding in what manner the system degenerates to attack self. By understanding the relationships between the immune system and distinguishing between self and non-self, there may be ways to protect the host from autoimmune diseases or diagnose those individuals who may be susceptible to autoimmune diseases and for whom there may be ways to provide some degree of protection.

Reports concerning treatment of a variety of autoimmune diseases, particularly with antibodies, include Steinman et al., Proc. Natl. Acad. Sci. USA (1981) 78:7111-7114; Zamvil et al., J. Exp. Med. (1985) 162:2107-2124; Waldor et al., Science (1985) 227:415-417; Zamvil et al., Nature (1985) 317:355-358; Steinman et al., Ann. NY Acad. Sci. (1986) 475:274-284; Zamvil et al., Nature (1986) 324:258-260; Steinman, "Treatment of Autoimmune Disease with Monoclonal Antibodies" In New Horizons in Animal Models for Autoimmune Disease, Academic Press, 1987, pp. 277-288; Vladutiu and Steinman, Cell Immunol. (1987) 109:169-180.

Relationships between T-cell receptor variable regions and other characteristics are described in Winoto et al., Nature (1986) 324:679-682; Kappler et al., Cell (1987) 49:263-271; and Sinha et al., Science (1988) 239:1026-1029.

Sequences of various T-cell receptor subunits may be found in Yoshikai et al., J. Exp. Med. (1986) 164:90-103; Arden et al., Nature (1985) 316:783-787; Kimura et al., J. Exp. Med. (1986) 164:739; and Lai et al., Nature (1988) 331:543-546.

See also U.S. Patent No. 4,695,459, issued September 22, 1987, which describes the treatment of autoimmune diseases using anti-Leu3 antibody.

WO-A-86/06413 relates to a reagent capable of binding to T-cells and having specificity for a unique sequence within the variable region of the β chain of the T-cell receptor, the presence of increased number of T-cells carrying the unique sequence relative to the number of T-cells carrying the sequence present in a normal subject being associated with a specific disease.

The present invention provides a method for detecting the propensity in a human host for an autoimmune disease, said method comprising detecting the presence in a human genome of a locus encoding at least a portion of the variable region of a T cell receptor associated with the occurrence of the autoimmune disease.

The invention also provides a peptide comprising an amino acid sequence encoded by a locus of a T cell receptor variable region associated with the occurrence of an autoimmune disease.

The invention further provides a pharmaceutical composition for inhibiting lymphocyte mediated attack on human syngeneic cells associated with an autoimmune disease comprising a monoclonal antibody specific for an amino acid sequence encoded by a locus of a T cell receptor variable region associated with the autoimmune disease, in admixture with a pharmaceutically acceptable carrier, and also provides a pharmaceutical composition for inhibiting lymphocyte attack on human syngeneic cells associated with an autoimmune disease comprising a peptide of the invention in admixture with a pharmaceutically accepted carrier.

The invention further provides a monoclonal antibody specific for an amino acid sequence encoded by a locus of a T cell receptor variable region associated with an autoimmune disease, for use as a medicament, and also provides the use of a monoclonal antibody specific for an amino acid sequence encoded by a locus of a T cell receptor variable region associated with an autoimmune disease for the manufacture of a medicament for treatment of an autoimmune disease.

The invention also provided a peptide of the invention for use as a medicament, and further provides the use of a peptide of the invention for the manufacture of a medicament for treatment of an autoimmune disease.

It has now been found that there is a correlation between the presence of one or more loci of the T-cell receptor variable region and the propensity for autoimmune disease. Furthermore, in those hosts which express the T-cell receptors comprising the expression product of such loci, preventing binding of such T-cells to the target cells can provide prophylactic and/or therapeutic benefit.

The T-cell receptor has two subunits involved in binding, either α and β, or γ and δ. The variable regions associated with the subunits have a similar organization to those of the immunoglobulins, the β and γ subunits having a variable region which comprises exons associated with the V, D and J regions, while the α and δ subunits comprise exons encoding the V and J regions. By rearrangement of germline DNA, the exons are joined to the constant or conserved region and by subsequent splicing of the messenger RNA, an open reading frame is achieved which encodes the subunit. Depending on the particular genetic inheritance of the host, the variable region loci of the individual will be different. By determining those loci associated with a particular autoimmune disease, one can diagnose, prenatally or any time thereafter, a propensity for the particular autoimmune disease and at any time prior to or during the occurrence of symptoms of the disease, treat the host in a manner which inhibits the attack by the T-cells carrying the particular variable region against the target cells. The T-cell receptors, depending on the nature of the T-cell, bind to Class I or Class II major histocompatibility antigens. CD4 is associated with Class II MHC.

The autoimmune diseases of significant prevalence include multiple sclerosis, associated with destruction of myelin and glial cells, rheumatoid arthritis, associated with joint lesions, system lupus erythematosus (SLE), associated with deposition of autoantibodies and immune complexes, psoriasis, pemphigus vulgaris, juvenile onset diabetes, associated with destruction of beta cells in the islet of Langerhans, Sjögren's disease, thyroid disease, Hashimoto's thyroiditis, and myasthenia gravis, while there are many others.

The variable loci associated with a propensity for the disease may be identified in a number of different ways. One way is where an animal model exists. In this situation, one can identify in vitro the particular locus common to the animal model which responds to the presence of a target protein fragment on an antigen-presenting cell, where the T-cells are properly restricted by the Class II MHC of the antigen-presenting cells. The demonstration of expression of the common variable region locus associated with the disease propensity can be achieved by having monoclonal antibodies specific for the peptide expressed by the locus. Alternatively, one could perform Southerns, to demonstrate the presence of the locus in the genome, or Northerns to demonstrate the transcription of the locus. In this manner, one can rapidly define the common loci of the variable region(s) of the subunit(s) common to the disease.

A 100% correlation is not to be expected, nor will it be normally achieved. It will usually be satisfactory that in at least 60%, preferably in at least 70% of the hosts positive for the disease, the locus is present. Similarly, in fewer than about 50%, preferably in fewer than 30% of the hosts which do not present the symptoms of the disease, the locus is present. These percentages should be based upon a statistically significant number of hosts.

The diseases susceptible today to animal models include multiple sclerosis, which finds analogy in experimental allergic encephalitis in mice, Hashimoto's thyroiditis, which finds analogy in experimental autoimmune thyroiditis in mice, systemic lupus erythematosus which finds analogy in NZR/W mice, myasthenia gravis, which finds analogy in experimental myasthenia gravis in mice, juvenile diabetes, which finds analogy in the NOD mouse and the BB rat.

Where an animal is not available and one is interested in primates, particularly humans, a number of different techniques may be employed to identify the loci of interest. Lymphocytes may be isolated from a host which has the autoimmune disease. Macrophages may be isolated to serve as antigen-presenting cells autologous to the host T-cells. Small numbers of T-cells, generally fewer than 10⁵, preferably fewer than 10³, may be expanded and used with the macrophages and the antigen associated with the autoimmune response to identify T-cells which are activated by the presence of the antigen and antigen-presenting cells. cDNA may be prepared from the messenger RNA of the cells, in groups of cells which show activation, as demonstrated by interleukin-2 secretion, expansion of cells, or other indicia. The resulting cDNA may then be subtracted from the same cell population which has not been subjected to activation, so that residual cDNA may be correlated with expansion of formation of cells having the T-cell receptor of interest, as well as increased expression of the T-cell receptor of interest. The DNA may be identified employing a probe for the conserved region and the variable region sequenced. The variable region sequence may then be used to identify the genomic locus, if such locus has not been previously sequenced and reported.

Alternatively, one may identify B-cells as peripheral blood lymphocytes from the host, which bind to the target antigen associated with the particular autoimmune disease, by panning, affinity chromatography, magnetic separation, or the like, where the antigen is used as the ligand for identifying surface immunoglobulin which binds to the antigen. The B-cells may then be immobilized on the surface and used for affinity separation of T-cells which recognize the fragment of the antigen in the context of an MHC to which the T-cells are restricted. The resulting T-cell population enriched for T-cells associated with the disease may then be repetitively reiteratively enriched using a similar procedure. The T-cell enriched population may now be further enriched by employing antigen-presenting cells without affinity separation to provide expansion of the target T-cell population. The resulting cell population may then be used as an immunogen to produce monoclonal antibodies. The monoclonal antibodies may be screened with T-cells from hosts having the autoimmune disease to identify common antigenic sites. The cells having common antigenic sites may be screened for the particular exons associated with the variable regions of the different hosts, to identify common loci as diagnostic of the disease and involved in the expression of T-cell receptor involved with the etiology of the disease.

T-cells can also be enriched in the case of autoimmune brain diseases, like multiple sclerosis, by incubating T-cells with γ-interferon pulsed astrocytes, with or without myelin. The astrocytes will present myelin fragments in the context of MHC to T-cells. Astrocytes do not constitutively express MHC Class II molecules, but can be induced to do so with γ-interferon.

These techniques are well established and can be readily repeated providing for identification of the relevant exons associated with a particular autoimmune disease. So far as the availability of an animal model, the DNA sequence homologies of the mouse and human variable region families have been correlated, so that identification of the mouse variable region will directly identify the human locus.

Selection of the particular T-cell receptor variable region gene product may be approached in a variety of ways, depending upon the nature of the disease, different protocols or approaches may be employed.

For those disease such as myasthenia gravis, where the specific auto-antigen which is the target of autoreactive T-cells is known, for myasthenia gravis the acetylcholine receptor, T-cells may be grown with specificity for the disease-inducing auto-antigen. For myasthenia gravis, acetylcholine receptor-specific T-cells are grown following the methodology for expanding auto-antigen-specific T-cell clones as previously described (Zamvil et al., Nature (1985) 317:355-358; and Zamvil et al., Nature (1986) 324:258-260). Peripheral blood lymphocytes (PBL) are isolated from whole blood of myasthenic patients. Assays are performed at a relatively low concentration of cells, generally fewer than 10⁶, preferably fewer than about 5x10⁵, in an appropriate medium, e.g. RPMI supplemented with 10% autologous serum. Cells may then be stimulated with mammalian acetylcholine receptor or specific peptides known to stimulate T-cells of myasthenic individuals: thus, myasthenics who are HLA-DR3, DQw2, respond to acetylcholine receptor peptide p257-261 (L-L-V-I-V-E-L-I-P-S-T-S-S), while myasthenics who are HLA-DR5, DQw3, respond to p195-212 (D-T-P-Y-L-D-I-I-Y-H-F-V-M-Q-R-L-P-L).

The dosage is conveniently about 5-20 µg/ml of the peptide to stimulate cultures administered initially for about 5 days, preferably about 6 days or more. Viable cells are collected and about 5x10⁵ viable cells are stimulated with 3x10⁷ autologous antigen-presenting cells (irradiated with about 3300 Rad) and about 20 µg/ml acetylcholine receptor for a specific peptide. About 12 days later, viable cells are collected, e.g. on a Ficoll gradient, and cloned by limiting dilution at about 0.3 cells/well in the presence of about 5x10⁵ antigen-presenting cells and human interleukin-2. Cells from individual wells are expanded and grown in tissue culture flasks.

The T-cell receptor variable gene usage may then be determined by sequencing the mRNA or cDNA using the polymerase chain reaction and oligonucleotides as primers corresponding to the leader-V and V-J or V-D junctions, depending upon whether the α or β subunit is involved. The polymerase chain reaction is extensively described in Sinha et al., Science (1988) 239:1026; and Saiki et al., Nature (1986) 324:163. By comparing the identified V region with cells from other victims of the disease, one can establish that a particular sequence may be identified with the disease.

For those diseases where a specific auto-antigen has not been identified, or an alternative route is desired, one may rely on oligoclonality of T-cells in a restricted anatomic compartment characteristic of the disease, for instance the synovium in rheumatoid arthritis (Stamenkovic et al., Proc. Natl. Acad. Sci. USA (1988) 85:1179-1183); or the cerebrospinal fluid in multiple sclerosis (Hafler et al., Neurology (1987) 36:314). T-cells are isolated from the compartment and their T-cell receptor V-region gene expanded with the polymerase chain reaction and sequenced utilizing the primer oligonucleotides described above. Again, the resulting sequences may be compared with other patients, victims of the particular diseases, to demonstrate the generality of the sequence as a diagnostic for the disease.

For those diseases, such as Graves disease, where the target organ is inaccessible except by surgical intervention, the association between the T-cell receptor V-region gene in the germline and susceptibility to the disease may be established as follows. By utilizing probes for the T-cell receptor and Southern hybridization, a T-cell receptor polymorphism may be identified in patients with the disease as contrasted with normal patients. When such polymorphisms are significantly associated with the disease (p ≤ 0.03 on X² analysis) the particular V-region gene product, associated with the T-cell receptor probe, may be used as a target for therapeutic intervention. This type of analysis has shown a specific T-cell receptor polymorphism in 90% (9/10) of patients with Graves disease, compared to 39/70 healthy individuals (p < 0.001); 23/28 patients with multiple sclerosis, compared to 21/70 controls (p < 0.01); and for 14/17 patients with myasthenia gravis, compared to 21/70 controls (p < 0.01).

The subject analysis may be used by itself or in conjunction with analyses associated with MHC relationships. A large body of literature has been developed demonstrating that many autoimmune diseases have an association with a particular histocompatibility antigen profile. By having both the association with the T-cell receptor and the particular HLA or MHC restriction, both the propensity for the autoimmune disease and the presence of T-cells which function in the etiology of the disease may be detected.

For diagnosis, either the nucleic acid or antigen may be detected. For nucleic acid, cells may be isolated by any convenient means and by employing appropriate probes, and using techniques such as Southern transfer, dot-blots, or the like, the presence of the particular V-region gene may be detected. Thus, one can readily determine whether there is a propensity for the autoimmune disease. Depending upon the nature of the disease, there may be an opportunity for prophylactic intervention to reduce the potential for the disease occurring. Alternatively, one may wish to determine the number of cells which are expressing the T-cell receptor. This can be achieved in one of two ways. The messenger RNA may be isolated from T-cells and probed with an appropriate probe for the V gene region. By employing Northern techniques, one can detect the presence of the messenger encoding the T-cell receptor and obtain a qualitative value for the amount of T-cell receptor being expressed containing the particular V-region gene.

For diagnosis, restriction fragment length polymorphisms (RFLP) may find application. Germline rearrangement of the diagnostic locus will provide a different sized fragment hybridizing with a probe for such locus, from unrearranged germline DNA. In this manner, a kit comprising a collection of probes for the loci diagnostic for an autoimmune disease may be employed for detection of size separated genomic fragments digested with one or two restriction enzymes. The probes may be labeled with radioactive labels, e.g. ³²P, biotin, fluorescers, or the like, for detection.

More conveniently, antibodies may be employed which may be prepared to be specific for the V region of a T-cell receptor of interest. Antibodies may be prepared for the α-V region and/or the β-V region and in some instances may be prepared for δ- or γ-V regions. The significant factor is that one can show a correlation between the presence of a particular V region and a propensity for or existence of a particular autoimmune disease.

The antibodies may be prepared in accordance with conventional ways, particularly employing the monoclonal antibody techniques, such as originally described by Kohler and Milstein, and as has been extensively improved for production of monoclonal antibodies. See, for example, U.S. Patent Nos. 4,690,893; 4,713,325; 4,714,681; 4,716,111; 4,716,117; and 4,720,459.

For diagnosis, any of a number of convenient techniques may be employed, which employ antibodies. Numerous patents as well as publications have described a number of techniques, using a wide variety of labels for detection. The labels include particles, enzymes, chromophores, fluorophores, chemiluminescers, and the like. The particular label or technique which is employed is not critical to this invention, any convenient technique may be employed. The techniques may employ either competitive, or non-competitive methodologies, including sandwich methodologies, where one antibody will bind to the constant region of the T-cell receptor or other conserved region, while the other antibody will be specific for the V region of interest. The cells will usually be lysed to provide membrane-free proteins in accordance with conventional ways. Cellular debris may be removed and the protein extracted and harvested. Alternatively, intact cells may be employed and detected by fluorescence activated cell sorting or the like, where the T-cell population may be quantitatively determined.

For therapeutic purposes, there may be an interest in using human antibodies. Normally, one will not be permitted to immunize a human host with the T-cell receptor or fragment thereof to activate B-cells specific for the sequence of interest. However, there are alternatives, in that mice or other lower mammal may be immunized, the genes encoding the variable regions of the antibodies specific for the T-cell region of interest isolated and manipulated by joining to an appropriate human constant region. The resulting chimeric construct, comprising a mouse variable region and a human constant region may then be transformed into a microorganism or mammalian host cell in culture, particularly a lymphocyte, and the hybrid antibodies expressed. Of particular interest would be IgG constant regions. See, for example, EPA 85.305604.2. (EP-A-0 173 494).

In some instances, it may be satisfactory to use mouse antibodies, where tolerance can be achieved or some degree of immune suppression may be involved. Immune suppression may be achieved with cyclosporin, irradiation, anti-Leu3 (anti-CD4)(U.S. Patent No. 4,681,760), or the like.

The antibodies may be used in a variety of ways, for example, for inhibiting binding between the T-cell and the target cell, for killing of T-cells, or for isolating the T-cells. In the first situation, the entire antibody may be administered, or Fab fragments, or even only the Fv region. By removing all or a portion of the constant region, there may be a reduction in the immune response. For selectively killing the T-cells carrying the particular V region, one may use a variety of immunotoxins, which may include the antibody or specific binding fragment thereof bonded to all or a portion of a plant toxin, such as ricin, abrin, etc., or diptheria toxin. By employing an appropriate antibody isotype, e.g. IgM or IgG₃, the complement cascade may be enlisted. Alternatively, a radioactive substituent may be used which provides for a lethal dosage upon binding of the antibody to the host cell. Alternatively, the antibody or fragment thereof may be conjugated with a cytolytic agent for specific elimination of the undesired T-cells. Finally, the T-cells can be removed by extracorporeal means, such as plasmaphoresis, where the plasma may be passed through or over antibodies bound to a support, with the undesired T-cells being selectively removed.

For therapeutic purposes, the antibody may be formulated with conventional pharmaceutically or pharmacologically acceptable vehicles for administration, conveniently by injection. Vehicles include deionized water, saline, phosphate-buffered saline, Ringer's solution, dextrose solution, and Hank's solution. Other additives may include additives to provide isotonicity, buffers, preservatives, and the like. The antibody or derivative thereof will usually be formulated in purified form at concentrations in the range of about 0.05 to 10 µg/ml. The antibody may be administered parenterally, typically intravenously or intramuscularly, as a bolus, intermittently or in a continuous regimen.

Desirably, the dose should deplete or at least bind about 75% of the undesired T-cells, preferably at least about 90%. Typical doses for adult humans will be in a range of about 10 to 100 mg. Doses for children or other animal species may be extrapolated from the adult human dose based on relative body weight.

Instead of antibodies, oligopeptides may be employed, having the same or substantially the same sequence as the oligopeptide sequence identified as being diagnostic of the autoimmune disease. These sequences will be oligopeptides of at least 8, usually at least 10 and preferably at least 12 amino acids, and generally not more than about 20 amino acids of the T-cell receptor subunit chain. While the entire subunit(s) may be employed, usually, not more than about 50 number % of the amino acids will be employed, particularly excluding the conserved or constant region, while all or a portion of the variable region including the particular locus will be included. The oligopeptide may be joined to other peptides or proteins for a variety of reasons, such as to provide for enhanced stability, tolerization, ease of preparation or purification, or the like. The subject peptides may be used to inhibit the binding of the T-cell receptor to the target antigen. The peptide may be formulated in substantially the same manner as described for the antibodies. The amount of active ingredient administered will vary widely depending on the particular composition, the particular host, the number and frequency of administrations, the manner of administration, etc. Usually there will be from about 0.01 to 10 µg/kg of host, more usually from about 0.05 to 5 µg/kg of host, where the concentration may range from about 10 µg/ml to about 1 µg/ml.

The manner of administration may be varied widely, depending upon the formulation and nature of the active ingredient. Administration may be parenteral, intravascular, peritoneally, subcutaneous, oral, etc., may employ catheters, pumps, constant diffusion membranes, etc.

The oligopeptides may be prepared in a variety of ways. Conveniently in accordance with conventional synthetic procedures. Where larger sequences are involved, such as 30 amino acids or more, recombinant DNA techniques may be employed, where the gene may be synthesized, in accordance with conventional ways, such as commercially available DNA synthesizers, expanded employing the polymerase chain reaction, and then inserted into an appropriate vector having the necessary transcriptional and translational initiation and termination regions. The resulting vector is then transformed into a host in which the expression vector is replicated and functional expression is obtained. The product may be secreted and harvested from the medium or when not secreted and retained cytoplasmic ally, the cells are harvested, lysed, and the desired protein isolated and purified in accordance with conventional ways.

Instead of the oligopeptide, anti-idiotype antibodies may be employed. By preparing a monoclonal antibody to the idiotypic epitope of the antibody to the subject oligopeptide, the anti-idiotype antibody may mimic the oligopeptide and serve to compete for the MHC with the T-cell receptor for the MHC. The anti-idiotype may provide greater stability on administration, as well as other advantages.

The protective compositions may be used in vitro or in vivo by adding to groups of cells comprising lymphocytes and cells associated with the autoimmune disease or target protein. By adding the protective composition, usually a protein such as an antibody or peptide having the appropriate variable region sequence, one can prevent the distribution of the cells and/or target protein. Where cells are involved, the T-cells will be restricted by the major histocompatibility antigen of the target cells, the target cells usually being syngeneic with the T-cells.

In addition to the compositions of this invention, other compositions may be employed to enhance the protection. These compositions may comprise oligopeptides, one or more different oligopeptides, comprising the following sequence: charged amino acid, two hydrophobic amino acids, and at least one of the next two amino acids being a polar amino acid, where the charged or polar amino acid may be substituted by glycine, usually not more than one being substituted by glycine. The charged amino acids are aspartic acid, glutamic acid, lysine, arginine, and histidine (D, E, K, R, H). The hydrophobic amino acids are alanine, proline, valine, leucine, isoleucine, methionine, phenylalanine, tryptophan, and tyrosine, that is both the aliphatic and aromatic neutral or substantially neutral amino acids having not more than one heteroatom, e.g., chalcogen, on the side chain (A, P, V, L, I, M, F, W, and Y). The polar amino acids will be the charged amino acids, as well as serine, threonine, asparagine, and glutamine (S, T, N, and Q).

The motif sequence will be part of a sequence of an immunogen of interest, associated with an autoimmune disease, e.g. myelin and multiple sclerosis, there usually being more than one partial sequence in the immunogen including the subject motif. The oligopeptide comprising the subject motif may be from any site of the immunogen sequence, that is N-terminal or C-terminal proximal or central, where the oligopeptide sequence will normally be substantially homologous with from 9 to 15 amino acids of the immunogen sequence, although longer sequences may also be employed. Usually the difference in homology will not be more than 2 non-conservative lesions, more usually not more than 2 lesions, which may be insertions, deletions, conservative or non-conservative substitutions.

Usually, the motif sequence present in the oligopeptide will be at other than the C-terminus of the oligopeptide, desirably being at the N-terminus and not closer to the C-terminus than the center of the sequence, where the second, third, or fourth amino acid of the motif (depending upon whether there are four or five amino acids in the motif) is the central amino acid.

The compositions of this invention will include usually at least one sequence of an immunogen of interest including the subject motif and may include two or more oligopeptide motif containing sequences of from about 9 to 15 amino acids present in the immunogen, depending upon the number of motifs present in the immunogen. Thus, if there are a plurality of motifs present in the immunogen, all or fewer than all of the sequences including the motifs may be employed in a single composition. Usually, there will be not more than 10 different motif comprising oligopeptides, more usually not more than about 6 different oligopeptides in the composition.

In preparing the subject compositions, one would select an immunogen related to the autoimmune disease of interest against which an immune response of a host is to be modulated. The oligopeptide may serve to tolerize the host to prevent immune attack against the endogenous protein or cell producing the endogenous protein.

The particular protein of interest will be screened for the presence of the subject motif and one or more sequences including the motif selected. Where the haplotype of the intended recipient is known, one sequence may be preferred over another. However, where the haplotype is not known, or the composition may be administered to a number of different hosts, it will frequently be desirable to combine a number of the sequences as oligopeptides in the same composition. The oligopeptides may be present as the individual peptides, or may be joined together in a single sequence, with or without intervening bridges, where any bridges will be other than the naturally occurring intervening sequences of the immunogen. Desirably, any such sequence would have fewer than about 100 amino acids, more usually fewer than about 60 amino acids.

The subject oligopeptides may be modified in a variety of ways. For tolerization, the subject peptides may be conjugated to syngeneic spleen cells, or be linked to an innocuous immunogen to which the host has been previously immunized, such as tetanus toxoid, bovine serum albumin, etc. Adjuvants are normally avoided.

Sequences which may be employed for tolerization will be sequences from proteins endogenous to the host involved with autoimmune diseases, which include such proteins as the neurological proteins found in the peripheral nervous system (PNS) or the central nervous system (CNS) and the acetylcholine receptor (AChR). These proteins are designated as Pₒ which is found in the PNS and CNS, P1, in myelin basic protein, the predominant CNS protein of myelin, P2, a predominant PNS myelin protein, PLP, a proteolipid protein, a PNS and CNS myelin constituent, and the acetylcholine receptor. P1 is involved in post-immunization encephalomyelitis and may be involved in multiple sclerosis. P2 is involved in post-immunization neuritis (Guillain-Barre syndrome) a major complication, for example, in the swine flu immunization program and the acetylcholine receptor is involved in myasthenia gravis and may play a role in post-immunization myositis. These protein fragments may be modified by one or more lesions to maintain the binding affinity for the transplantation antigen while substantially reducing the binding affinity to the T-cell receptor.

In addition to oligopeptides including the subject motif, other oligopeptides which may be employed are those N-terminal peptides which are acetylated, where the only requirement is that the parent immunogen is present as the N-acetylated protein. Of particular interest for this purpose is the N-terminus of myelin basic protein, which has the sequence Ac-A-S-Q-K-R-P-S-Q-R-H-G-S-K-Y-L, other acetylated N-termini include the P2 protein, which has the sequence Ac-S-N-K-F-L-G-T-W-K-L-V-S-S-G.

Transplantation antigens have polymorphic regions, where the individual alleles are associated with specific hosts. For the most part, the host will be diploid and heterozygous, so that each host will have two haplotypes, meaning that there will be two different copies of a particular transplantation antigen type from the same locus, unless the host is homozygous at that particular locus. Therefore, as to an individual host or a plurality of hosts, mixtures of oligopeptides will usually be employed.

The subject oligopeptides may be administered concurrently or consecutively with the oligopeptides of the T-cell receptor. The subject oligopeptides may be administered in a variety of ways, by themselves or in conjunction with various additives. Various carriers may be employed which are physiologically acceptable, such as water, alcohol, saline, phosphate buffered saline, sugar, mineral oil, etc. Other additives may also be included, such as stabilizers, detergents, flavoring agents, thickeners, etc. The amount of active ingredient administered will vary widely depending upon the particular composition, the particular host, the number and frequency of administrations, the manner of administration, etc. Usually, there will be from about 0.01 to 10 µg/kg of host more usually from about 0.05 to 5 µg/kg of host, where the concentration may range from 10 µg/ml to 1 mg/ml.

The following examples are offered by way of illustration and not by way of limitation.

### EXPERIMENTAL

### MATERIAL AND METHODS

### Mice.

PL/J and (PL/J X SJL/J)F₁ ([PLSJ]F₁) female mice were purchased from the Jackson Laboratory, Bar Harbor, ME, and housed in the animal facilities in the Departments of Genetics and Medical Microbiology, Stanford University, Stanford, CA.

### Antigens.

NH₂-terminal MBP peptides were synthesized according to the sequences for rat and bovine MBP by solid phase techniques (Zamvil et al., Nature (1986) 324:258). These peptides contained >90% of the desired product as determined by a high-pressure liquid phase column (Merck & Co., Inc., Rahway, NJ) and amino acid analysis.

### T-cell Clones.

NH₂-terminal MBP-specific T-cell clones were isolated using intact MBP and MBP peptides (Zamvil et al., ibid (1985) 317:355; Zamvil et al., J. Exp. Med. (1985) 162:2107). These clones were isolated from homozygous PL/J mice, except clones F₁-12 and F₁-21, which were isolated from a (PLSJ)F₁ mouse. All clones described share the same Class II (I-A^{u}) restriction and NH₂-terminal MBP (1-9) specificity. Clones isolated from the same T-cell line differed in their TCR β chain rearrangement or their reactivity to TCR V_{β8}-specific mAbs (Table I).

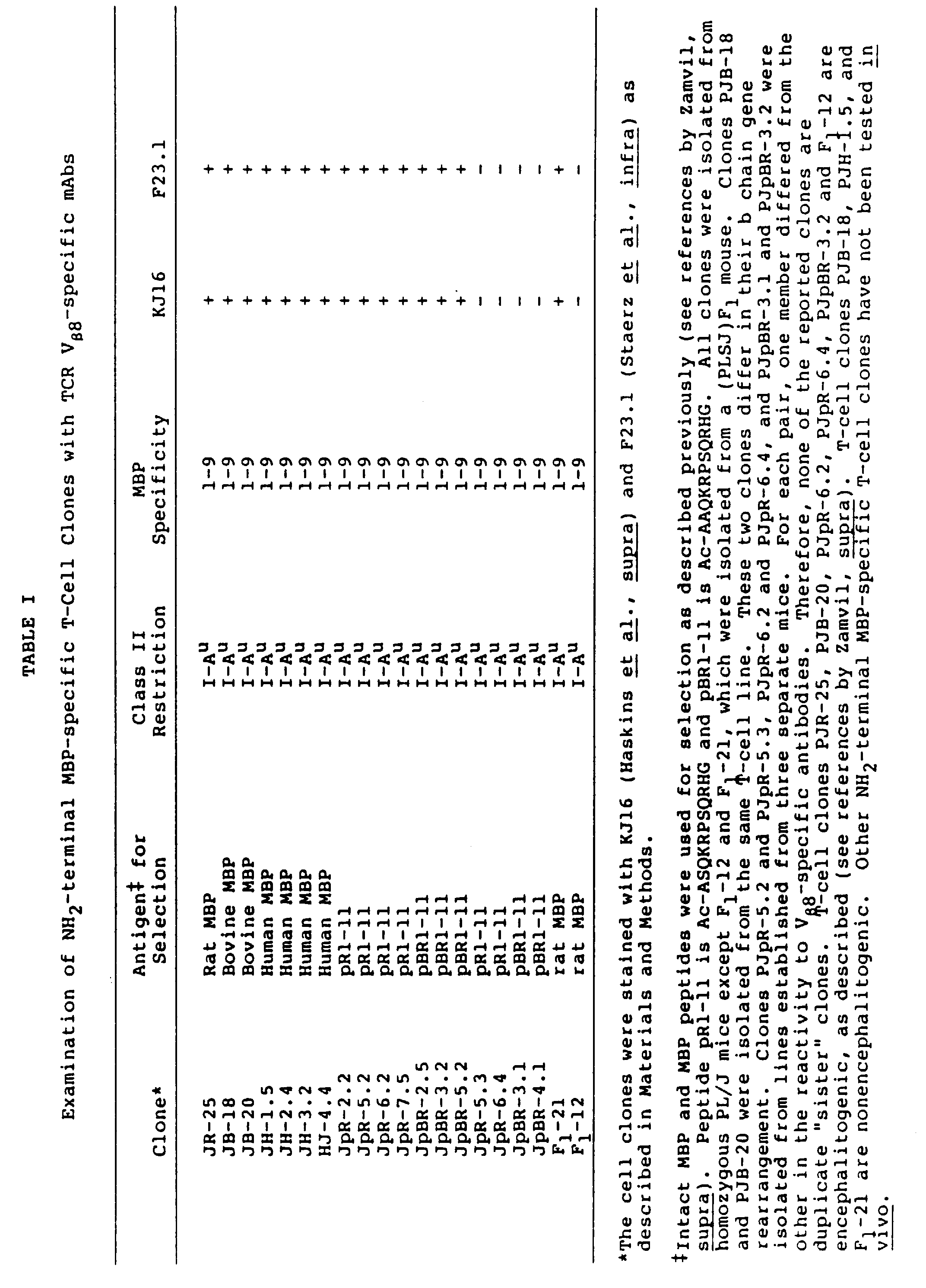

Thus, none of the clones discussed are duplicate "sister" clones. T-cell clones PJR-25, PJB-20, PJpR-6.2, PJpR-6.4, PJpBR-3.2, and F₁-12 are encephalitogenic. T-cell clones PJB-18, PJH-1.5, and F₁-21 are nonencephalitogenic (Zamvil et al., J. Immunol. (1987) 139:1075). Other NH₂-terminal MBP-specific T-cell clones have not been tested in vivo.

### Proliferation Assay.

The proliferative responses were determined as described in Zamvil et al., Nature (1985), supra. Either 10⁴ cloned T-cells or 4x10⁴ lymph node cells were cultured with 5x10⁵ γ-irradiated (3000 rad) PL/J splenic APC in 0.2 ml culture media in 96-well flat-bottomed microtitre plates (model 3072; Falcon Labware, Oxnard, CA). At 48 h of incubation, each well was pulsed with 1 µCi[³H]thymidine and harvested 16 h later. The mean cpm thymidine incorporation was calculated for triplicate cultures. Standard deviations from replicate cultures were within 10% mean value.

### FACS Analysis.

Each clone was stained with KJ16 (Haskins et al., J. Exp. Med. (1984) 160:452) and anti-L3T4 (GKl.5) (Dialynas et al., Immunol. Rev. (1983) 74:29; Hayakawa et al., J. EXP. Med. (1983) 157:202). 5x10⁵ T-cells were incubated with 1 µg biotin-conjugated KJ16 and 1 µg fluorescein-conjugated GK1.5, followed by Texas red-avidin. Immunofluorescence analysis was performed on a dual laser FACS IV (Becton Dickinson Immunocytometry, Fullerton, CA) equipped with logarithmic amplifiers. Two-color staining data were obtained as "contour plots", in which the levels of green and red fluorescence per cell defined the location on a two-dimensional surface.

### Southern Analysis.

TCR probe V_{βc5.1} is a 280-bp Pst-Pvu II subclone of V_{β8.1}(V_{βc5.1}) (Patten et al., Nature (1984) 31240). DNA probe 5'V_{βc5.2}, a 0.5-kb Pvu II fragment (available from M.M. Davis, Stanford University, Stanford, CA), located 2 kb 5' of V_{β8.2}. J_{β2}-specific probe is a 1.2-kb genomic probe encompassing J_{β2.1 2.7} (Chien et al., Nature (1984) 309:322). Liver and T-cell DNA were isolated with standard methods (Kaiser and Murray, 1985, In DNA Cloning: A Practical Approach, Vol. I, M. Glover, ed., IRL Press Limited, Oxford, pp. 38-39). Each sample, containing 10 µg DNA, was digested to completion with either Xba I or Hind III, separated on 0.8% agarose gels, and transferred to nitrocellulose filters. The filters were prehybridized at 42°C for 4 h in 50% formamide, 5X SSPE, 100 µg/ml salmon sperm DNA, 1X Denhardt's solution, 0.1% SDS, and 10 mM Tris (pH 8.0). Hybridizations were performed in the same buffer containing 5x10⁶ cpm/ml of hexamer-primed probe for 20 h at 42°C. The filters were washed in 2X SSC, 0.1% SDS at room temperature. They were washed two more times for 1 h each at 60°C in 0.2X SSC, 0.1% SDS.

### Induction of EAE.

Induction of EAE with encephalitogenic MBP-specific T-cell clone PJR-25 was performed as described in Zamvil et al., Nature (1985), supra; Zamvil et al., J. Exp. Med. (1985), supra. 5x10⁶ Ficoll-separated cells of clone PJR-25 were injected intravenously into recipient (PLSJ)F₁ mice 6 h after intraperitoneal injection of mAb. EAE was graded as described previously in Zamvil et al., Nature (1985), supra; Zamvil et al., J. Exp. Med. (1985), supra: 0, no sign of EAE; 1, decreased tail tone only; 2, mild paraparesis; 3, moderately severe paraparesis; 4, complete paraplegia; 5, moribund.

### RESULTS

### TCR β Chain Expression of NH₂-terminal MBP-specific T-cells.

Twenty myelin binding protein (MBP)-specific T-cell clones (L3T4⁺, Lyt2⁻) were examined in this study. Eighteen of these clones were isolated from 14 individual homozygous PL/J mice, and two clones were isolated from a single (PLSJ)F₁ mouse. These clones were isolated after immunization with different forms of MBP and MBP peptides. All 20 clones share the same NH₂-terminal nonapeptide specificity and Class II (I-A^{u}) restriction. They are not alloreactive for foreign MHC Class II molecules. TCR β chain gene expression of these clones was examined with mAbs specific for the TCR V_{β8} gene subfamily. The TCR-specific mAb KJ16.133 (Haskins et al. (1984), supra) recognizes a determinant associated with expression of two members of this TCR V_{β} gene subfamily (Behlke et al., J. Exp. Med. (1987) 165:257) (also known as V_{βc5} (Patten et al., supra), V_{β8.1} (V_{βc5.1}), and V_{β8.2} (V_{Bc5.2}). The KJ16 phenotype is expressed by 16% of PL/J T lymphocytes. In csntrast, SJL/J mice do not express the V_{β8} (KJ16) subfamily. The FACS-staining pattern with KJ16 for six representative T-cell clones, four of which are KJ16⁺ and two of which are KJ16⁻ was determined. Of the 18 PL/J clones, 14 (78%) are KJ16⁺ (Table I). This high percentage of KJ16⁺ (V_{β8}⁺) clones is not an artifact from examining duplicate sister clones isolated from the same T-cell line. For those lines where two clones are shown (Table I), the clones differed either in their antibody reactivity or their TCR β chain gene rearrangement. Thus 78% KJ16⁺ clones is a minimum. When additional NH₂-terminal MBP-specific sister clones were examined, 85% are KJ16⁺(V_{β8}⁺). Furthermore, the KJ16⁻ clones are not stained with another mAb, F23.1 (Staerz et al., J. Immunol. (1985) 134:3994), which recognizes a TCR determinant associated with the expression of all three members of the V_{β8} subfamily, V_{β8.1}, V_{β8.2}, and V_{β8.3} (Behlke et al., supra).

The predominant use of the TCR V_{β8} gene subfamily is not an in vitro cloning artifact. The proliferative T-cell response was examined from MBP 1-11 (1-11 amino acids of MBP)-primed PL/J mice after FACS sorting L3T4⁺ lymph node cells into L3T4⁺/V_{β8}⁺ and L3T4⁺/V_{β8}⁻ subpopulations. In two separate experiments, >90% of the proliferative T-cell response occurs in the V_{β8}⁺ subpopulation. Although the V_{β8}⁻ subpopulation of MBP 1-11-primed lymph node cells does not respond to MBP 1-11, this subpopulation does respond to PPD (purified protein derivative of mycobacterium Tuberculosis), a positive control. Thus, by examining both in vitro isolated T-cell clones and primary cultures, it was shown that there is predominant usage of the TCR V_{β8} subfamily in the autoimmune T lymphocyte response to the NH₂ terminus of MBP.

### Genetic Analysis of TCR β-Chains of NH₂-terminal MBP-specific T-Cell Clones.

Southern blot analysis was used to determine whether NH₂-terminal MBP-specific KJ16⁺ T-cell clones use the same β chain V-D-J combination and to identify which member(s) of the V_{β8} subfamily is used. A blot of Xba I digested genomic DNA isolated from several of these clones was screened with a probe, designated V_{βc5.1}, which is specific for the V_{β8}(V_{βc5}) subfamily. This probe hybridizes to all three V_{β8} genes, V_{β8.1} being 92 and 85% homologous to V_{β8.2} and V_{β8.3}, respectively (Barth et al., Nature (1985) 316:517). On germline DNA digested with Xba I, V_{β8.1}, V_{β8.2}, and V_{β8.3}, genes are located on separate 10.0-, 5.5- and 3.9-kb fragments, respectively. It was observed that there were rearrangements of a V_{β8} subfamily member of KJ16⁺ clones PJR-25, PJB-18, and PJpR-6.2. A germline configuration for all V_{β8} members is observed for the KJ16⁻ clone PJpR-6.4. The sizes of these rearranged bands are consistent with all the KJ16⁺ clones using V_{β8.2}. To confirm this possibility, a blot of Xba I digested DNA was also probed with a 0.5-kb Pvu II fragment (available from M.M. Davis, Stanford University, Stanford, CA) located 2 kb 5' to V_{β8.2}. A rearrangement detected using this 0.5-kb probe is specific for the use of V_{β8.2}. Rearrangement of the V_{β8.2} gene is observed for all four KJ16⁺ clones examined by hybridization with this 0.5-kb probe. Another KJ16⁺ clone, encephalitogenic clone PJpBR-3.2 (Table I), which was examined in the same manner also uses TCR V_{β8.2}.

The sizes of the fragments seen on DNA digested with Xba I are consistent with the use of members of the J_{β2} locus. To confirm this, blots were made from DNA digested with Hind III. V_{β8.2} and V_{β8.3} are both located on the same 9.6-kb Hind III fragment. V_{β8.1} is located on a 1.2-kb fragment. Using V_{β8.2}, a rearrangement involving J_{β1} produces a Hind III fragment of 13-14 kb, whereas rearrangement involving J_{β2} produces a Hind III fragment of 8-10 kb. Blots made from DNA digested with Hind III were hybridized with the probe V_{βc5.1}(V_{β8.1}) and a J_{β2}-specific probe (Chien et al., ₛᵤₚᵣₐ). The 1.2-kb fragment containing V_{β8.1} migrated off these gels. The rearranged bands seen by hybridization with both J_{β2} and V_{βc5.1}(V_{β8.1}) probes are 8.5-9.5 kb, indicating that each rearrangement of V_{β8.2} is a V_{β8.2}DJ_{β2} rearrangement. Two patterns of rearrangements were seen with the Vβ and Jβ probes indicating that two different Jβ genes at the second locus must be used. Results of an EcoRV blot indicated the use of two separate Jβ genes. The rearrangement of clone PJR-25 is like that of PJB-18 and the rearrangement of clone PJB-20 is the same as PJpR-6.2.

T-cell clone PJB-18 has TCR β chain rearrangements involving the V_{β8} locus on both homologous chromosomes. One rearrangement uses V_{β8.2} and a separate rearrangement is consistent with the use of V_{β8.3}. The two V-D-J rearrangements resulted in the deletion of both germline V_{β8.1} and V_{β8.2} bands. T-cell clone PJpR-6.2 deleted all of the members of the V_{β8} gene subfamily on one chromosome and has a rearrangement to V_{β8.2} on the other, leaving one germline copy of the V_{β8.3} gene on that chromosome. With a J_{β1}-specific probe, it was shown that this clone uses a J_{β1} gene for the V-D-J rearrangement on the other chromosome. Both chromosomes can rearrange TCR genes, though in accordance with allelic exclusion, only one is productive. Of the two rearrangements for PJB-18 and PJpR-6.2, the V_{β8.2}-containing rearrangement must be the functional one because both clones react with KJ16, which recognized V_{β8.1} and V_{β8.2}, but not V_{β8.3}. One other V_{β} family has been identified in clones specific for the N-terminus of myelin binding protein with the same Class II restriction, V_{β4}, as a minor contributor.

### Prevention of EAE with Anti-TCR T-Cell Receptor V Region Antibody

Recipient (PLSJ)F₁ mice were injected intraperitoneally with 500 µg of DEAE column-purified monoclonal antibody F23.1 which recognizes a TCR determinant associated with the expression of all three members of the V_{β8} subfamily (Staerz et al., supra). Control mice were injected intraperitoneally with 1.0 ml PBS. Six hours later, recipient mice were injected with 5x10⁶ cells of encephalitogenic clone PJR-25. Mice were examined daily by two independent observers. Severity of EAE was graded as follows: 0, no sign of EAE; 1, decreased tail tone only; 2, mild paraparesis; 3, moderately severe paraparesis; 4, complete paraplegia; 5, moribund. Results of this experiment are given in the table below.

**TABLE II**

| In vivo Administration of an mAB Specific for the Antigen-specific TCR Prevents Induction of EAE | | | | |
|---|---|---|---|---|
| Exp. | F23.1 | Incidence | Severity | Day of Onset |
| 1 | + | 0/6 | -- | -- |
| | - | 6/6 | 3.5 | 42 |
| 2 | + | 0/5 | -- | -- |
| | + | 0/5∗ | -- | -- |
| | - | 5/5 | 4.0 | 27 |

| | | | | |
|---|---|---|---|---|
| ∗Injected with 500 µg mAB F23.1 14 days after injection of PJR-25. | | | | |

In contrast, administration of F23.1 to SJL/J mice which lack the V_{β8} subfamily, does not prevent EAE. When KJ23a (Kappler et al., Cell (1987) 49:263) is administered to SJL/J mice given a T-cell line reactive to myelin basic protein peptide p89-101, 0/4 mice developed EAE compared to 10/12 given F23.1. KJ23a recognizes V_{β17a}. V_{β17a} is homologous to V_{β4} in man, while Vβ8.2 is homologous to V_{β12} in man (Lai et al., Nature (1988) 331:543).

In the next study, it was determined whether EAE could be prevented when (PLSJ)F₁ mice were given myelin basic protein peptide p1-11 in complete Freund's adjuvant (CFA). p1-11 in CFA induces T-cell clones that utilize the V_{β8.2} TCR gene family as well as clones that recognize p1-11 and do not rearrange V_{β8.2}. Seventy-eight percent (14/18) T-cell clones reactive to p1-11 rearrange V_{β8.2} and can be stained with monoclonal antibodies KJ16 and F23.1 on flow cytometric analysis. Mice were given 500 µg intraperitoneally of F23.1 one day before, one day after, and nine days after treatment with p1-11 in complete Freund's adjuvant. Control mice received Leu 5b, a non-cross reactive mouse monoclonal that is isotype matched for F23.1. Whereas 1/19 mice receiving F23.1 developed EAE, 10/20 mice given Leu 5b developed EAE and became paralyzed (p < 0.001). These results serve to indicate that V_{β8.2} is critical in the development of EAE, a model for multiple sclerosis, and that escape to V_{β8.2} negative clones that could produce EAE, does not occur.

### Reversal of EAE with Anti-TCR V Region Antibody

EAE was induced with T-cell clone PJr25 as described previously (Zamvil et al., Nature (1986) 324:258). Therapy was initiated with mAb F23.1 within 24 h of the time mice first developed paralysis. (PLSJ)F1 mice were randomized into two groups with 16 receiving F23.1 and 16 paralyzed mice receiving Leu 5b, an isotype matched control reactive with the CD2 antigen. Mice were given 2 injections of monoclonal antibody (100 µg) first within 24 h of the onset of paralysis and then 72 h later. Within 96 h of initiation of treatment with F23.1, mice showed a marked reversal in their paralysis and 13 of 16 were completely free of disease 10 days after therapy commenced. Control animals remained paralyzed during the 40 days of observation after therapy started. One relapse was seen on day 35 in the animals given mAb F23.1 Similar results were seen when EAE was induced in (PLSJ)F1 mice with myelin basic protein in complete Freund's adjuvant.

After paralysis was present, mice were given 0.2 mg intraperitoneally of F23.1 (anti-V_{β8.2}) or KJ23a (anti-V_{β17a}). KJ23a prevents EAE induced with V_{β17a} positive T-cells in SJL/J mice given myelin basic protein p89-106. Twelve of 19 mice given F23.1 returned to normal within 72 h, while 21 out of 22 mice given KJ23a continued to be paraplegic after 72 h. Relapses occurred in six out of 19 F23.1-treated mice over the next two weeks, while 21 out of 22 KJ23a-treated mice remained paralyzed.

These results indicate that there is a restricted TCR β chain expression in the autoimmune response to the encephalitogenic NH₂ terminus of myelin basic protein. There is a predominant expression of the TCR V_{β8} subfamily, in particular V_{β8.2}. These results further indicate that in vivo administration of a TCR V_{β8}-specific monoclonal antibody can prevent or reverse EAE, a model of multiple sclerosis. Thus, in clinical situations where autoaggressive T-cells can be identified, antibodies to the TCR V region can provide a highly specific form of therapy.

It is evident from the above results, that the subject invention provides a novel insight, which can be used for diagnosis, prophylaxis and treatment of autoimmune diseases. Thus, sequences can be identified which can indicate the propensity of an individual for a particular autoimmune disease, where early intervention may prevent or significantly reduce the deteriorating effects of the disease. Thus, opportunities exist for prophylaxis and therapy in such debilitating diseases as multiple sclerosis, SLE, diabetes, and the like. The method employed for diagnosis and treatment may follow well-established protocols and techniques where common reagents may be employed in conjunction with the materials provided in accordance with this invention. In this manner, the subject invention allows for ease in monitoring and treating the aforementioned autoimmune diseases.

Modifications of the above described modes for carrying out the invention that are obvious to persons of skill in medicine, immunology, hybridoma technology, pharmacology, and/or related fields are intended to be within the scope of the following claims.

## Claims (Claims for the following Contracting State(s): AT, BE, CH, DE, FR, GB, IT, LI, LU, NL, SE)

1. A method for detecting the propensity in a human host for an autoimmune disease, said method comprising detecting the presence in a human genome of a locus encoding at least a portion of the variable region of a T cell receptor associated with the occurrence of the autoimmune disease.

2. A method as claimed in claim 1, which comprises detecting nucleic acid encoding the portion of the variable region of the T cell receptor.

3. A method as claimed in claim 2, wherein the nucleic acid is genomic DNA.

4. A method as claimed in claim 1, which comprises detecting the expression product of the locus.

5. A method as claimed in any one of claims 1 to 4, wherein the locus encodes at least a portion of the variable region of a ß-subunit of a T cell receptor.

6. A method as claimed in claim 5, wherein the locus encodes at least a portion of a Vβ₁₂ or Vβ₄ region.

7. A method as claimed in claim 5, wherein the locus is the human equivalent of the mouse locus Vβ_{8.1}, Vβ_{8.2}, Vβ_{8.3} or Vβ₁₇ₐ.

8. A method as claimed in any one of claims 1 to 7, wherein the autoimmune disease comprises the destruction of glial cells and/or myelin.

9. A method as claimed in any one of claims 1 to 7, wherein the autoimmune disease is multiple sclerosis.

10. A method as claimed in any one of claims 1 to 5, wherein the autoimmune disease is rheumatoid arthritis, systemic lupus erythematosus, psoriasis, pemphigus vulgaris, juvenile onset diabetes, Sjögren's disease, thyroid disease, Hashimoto's thyroiditis or myasthenia gravis.

11. A peptide comprising an amino acid sequence encoded by a locus of a T cell receptor variable region associated with the occurrence of an autoimmune disease.

12. A peptide as claimed in claim 11, wherein the amino acid sequence comprises at least 8 amino acids.

13. A peptide as claimed in claim 11 or claim 12, wherein the locus encodes at least a portion of the variable region of a β-subunit of a T cell receptor.

14. A peptide as claimed in claim 13, wherein the locus encodes at least a portion of a Vβ₁₂ or Vβ₄ region.

15. A peptide as claimed in claim 13, wherein the locus is the human equivalent of the mouse locus Vβ_{8.1}, Vβ_{8.2}, Vβ_{8.3} or Vβ₁₇ₐ.

16. A peptide as claimed in any one of claims 11 to 15, wherein the autoimmune disease comprises the destruction of glial cells and/or myelin.

17. A peptide as claimed in claim 16, wherein the autoimmune disease is multiple sclerosis.

18. A peptide as claimed in any one of claims 11 to 13, wherein the autoimmune disease is rheumatoid arthritis, systemic lupus erythematosus, psoriasis, pemphigus vulgaris, juvenile onset diabetes, Sjögren's disease, thyroid disease, Hashimoto's thyroiditis or myasthenia gravis.

19. A pharmaceutical composition for inhibiting lymphocyte mediated attack on human syngeneic cells associated with an autoimmune disease comprising a monoclonal antibody specific for an amino acid sequence encoded by a locus of a T cell receptor variable region associated with the autoimmune disease, in admixture with a pharmaceutically acceptable carrier.

20. A pharmaceutical composition as claimed in claim 19, wherein the monoclonal antibody is a humanized antibody.

21. A pharmaceutical composition according to claim 19 or claim 20, wherein the monoclonal antibody is specific for an amino acid sequence encoded by a locus comprising at least a portion of the variable region of a β-subunit of a T cell receptor.

22. A pharmaceutical composition as claimed in claim 21, wherein the locus encodes at least a portion of a Vβ₁₂ or Vβ₄ region.

23. A pharmaceutical composition as claimed in claim 21, wherein the locus is the human equivalent of the mouse locus Vβ_{8.1}, Vβ_{8.2}, Vβ_{8.3} or Vβ₁₇ₐ.

24. A pharmaceutical composition as claimed in any one of claims 19 to 23, wherein the autoimmune disease comprises the destruction of glial cells and/or myelin.

25. A pharmaceutical composition as claimed in claim 24, wherein the autoimmune disease is multiple sclerosis.

26. A pharmaceutical composition as claimed in any one of claims 19 to 21, wherein the autoimmune disease is rheumatoid arthritis, systemic lupus erythematosus, psoriasis, pemphigus vulgaris, juvenile onset diabetes, Sjögren's disease, thyroid disease, Hashimoto's thyroiditis or myasthenia gravis.

27. A pharmaceutical composition for inhibiting lymphocyte attack on human syngeneic cells associated with an autoimmune disease comprising a peptide as claimed in any one of claims 11 to 18, in admixture with a pharmaceutically acceptable carrier.

28. A monoclonal antibody specific for an amino acid sequence encoded by a locus of a T cell receptor variable region associated with an autoimmune disease, for use as a medicament.

29. Use of a monoclonal antibody specific for an amino acid sequence encoded by a locus of a T cell receptor variable region associated with an autoimmune disease for the manufacture of a medicament for treatment of an autoimmune disease.

30. Use of a monoclonal antibody as claimed in claim 29, wherein the autoimmune disease comprises the destruction of glial cells and/or myelin.

31. Use as claimed in claim 30, wherein the autoimmune disease is multiple sclerosis.

32. Use of a monoclonal antibody as claimed in claim 29, wherein the autoimmune disease is rheumatoid arthritis, systemic lupus erythematosus, psoriasis, pemphigus vulgaris, juvenile onset diabetes, Sjögren's disease, thyroid disease, Hashimoto's thyroiditis or myasthenia gravis.

33. A peptide as claimed in any one of claims 11 to 18, for use as a medicament.

34. Use of a peptide as claimed in any one of claims 11 to 18, for the manufacture of a medicament for treatment of an autoimmune disease.

35. Use as claimed in claim 34, wherein the autoimmune disease comprises the destruction of glial cells and/or myelin.

36. Use of a peptide as claimed in claim 35, wherein the autoimmune disease is multiple sclerosis.

37. Use as claimed in claim 34, wherein the autoimmune disease is rheumatoid arthritis, systemic lupus erythematosus, psoriasis, pemphigus vulgaris, juvenile onset diabetes, Sjögren's disease, thyroid disease, Hashimoto's thyroiditis or myasthenia gravis.

38. An anti-idiotype antibody to a peptide as claimed in any one of claims 11 to 18.

## Claims (Claims for the following Contracting State(s): ES)

1. A method for detecting the propensity in a human host for an autoimmune disease, said method comprising detecting the presence in a human genome of a locus encoding at least a portion of the variable region of a T cell receptor associated with the occurrence of the autoimmune disease.

2. A method as claimed in claim 1, which comprises detecting nucleic acid encoding the portion of the variable region of the T cell receptor.

3. A method as claimed in claim 2, wherein the nucleic acid is genomic DNA.

4. A method as claimed in claim 1, which comprises detecting the expression product of the locus.

5. A method as claimed in any one of claims 1 to 4, wherein the locus encodes at least a portion of the variable region of a β-subunit of a T cell receptor.

6. A method as claimed in claim 5, wherein the locus encodes at least a portion of a Vβ₁₂ or Vβ₄ region.

7. A method as claimed in claim 5, wherein the locus is the human equivalent of the mouse locus Vβ_{8.1}, Vβ_{8.2}, Vβ_{8.3} or Vβ₁₇ₐ.

8. A method as claimed in any one of claims 1 to 7, wherein the autoimmune disease comprises the destruction of glial cells and/or myelin.

9. A method as claimed in any one of claims 1 to 7, wherein the autoimmune disease is multiple sclerosis.

10. A method as claimed in any one of claims 1 to 5, wherein the autoimmune disease is rheumatoid arthritis, systemic lupus erythematosus, psoriasis, pemphigus vulgaris, juvenile onset diabetes, sjögren's disease, thyroid disease, Hashimoto's thyroiditis or myasthenia gravis.

11. A method for producing a peptide comprising an amino acid sequence encoded by a locus of a T cell receptor variable region associated with the occurrence of an autoimmune disease, which comprises chemically synthesizing the peptide, or incubating a host cell transformed by a vector comprising a DNA sequence encoding the peptide and also comprising transcriptional and translational initiation and termination regions, under conditions that provide for expression of the coding sequence.

12. A method as claimed in claim 11, wherein the amino acid sequence of the peptide comprises at least 8 amino acids.

13. A method as claimed in claim 11 or claim 12, wherein the locus encodes at least a portion of the variable region of a β-subunit of a T cell receptor.

14. A method as claimed in claim 13, wherein the locus encodes at least a portion of a Vβ₁₂ or Vβ₄ region.

15. A method as claimed in claim 13, wherein the locus is the human equivalent of the mouse locus Vβ_{8.1}, Vβ_{8.2}, Vβ_{8.3} or Vβ₁₇ₐ.

16. A method as claimed in any one of claims 11 to 13, wherein the autoimmune disease comprises the destruction of glial cells and/or myelin.

17. A method as claimed in claim 16, wherein the autoimmune disease is multiple sclerosis.

18. A method as claimed in any one of claims 11 to 13, wherein the autoimmune disease is rheumatoid arthritis, systemic lupus erythematosus, psoriasis, pemphigus vulgaris, juvenile onset diabetes, Sjögren's disease, thyroid disease, Hashimoto's thyroiditis or myasthenia gravis.

19. A method for the production of a pharmaceutical composition for inhibiting lymphocyte mediated attack on human syngeneic cells associated with an autoimmune disease, which comprises admixing a monoclonal antibody specific for an amino acid sequence encoded by a locus of a T cell receptor variable region associated with the autoimmune disease, with a pharmaceutically acceptable carrier.

20. A method as claimed in claim 19, wherein the monoclonal antibody is a humanized antibody.

21. A method as claimed in claim 19 or claim 20, wherein the monoclonal antibody is specific for an amino acid sequence encoded by a locus comprising at least a portion of the variable region of a β-subunit of a T cell receptor.

22. A method as claimed in claim 21, wherein the locus encodes at least a portion of a Vβ₁₂ or Vβ₄ region.

23. A method as claimed in claim 21, wherein the locus is the human equivalent of the mouse locus Vβ_{8.1}, Vβ_{8.2}, Vβ_{8.3} or Vβ₁₇ₐ.

24. A method as claimed in any one of claims 19 to 21, wherein the autoimmune disease comprises the destruction of glial cells and/or myelin.

25. A method as claimed in claim 24, wherein the autoimmune disease is multiple sclerosis.

26. A method as claimed in any one of claims 19 25, wherein the autoimmune disease is rheumatoid arthritis, systemic lupus erythematosus, psoriasis, pemphigus vulgaris, juvenile onset diabetes, Sjögren's disease, thyroid disease, Hashimoto's thyroiditis or myasthenia gravis.

27. A method for the production of a pharmaceutical composition for inhibiting lymphocyte attack on human syngeneic cells associated with an autoimmune disease, which comprises admixing a peptide produced by a method as claimed in any one of claims 11 to 20, with a pharmaceutically acceptable carrier.

28. A method for the production of an anti-idiotype antibody to a peptide as defined in any one of claims 11 to 18, which comprises preparing a monoclonal antibody to the idiotype of the antibody to the peptide.

## Claims (Claims for the following Contracting State(s): GR)

1. A method for detecting the propensity in a human host for an autoimmune disease, said method comprising detecting the presence in a human genome of a locus encoding at least a portion of the variable region of a T cell receptor associated with the occurrence of the autoimmune disease.

2. A method as claimed in claim 1, which comprises detecting nucleic acid encoding the portion of the variable region of the T cell receptor.

3. A method as claimed in claim 2, wherein the nucleic acid is genomic DNA.

4. A method as claimed in claim 1, which comprises detecting the expression product of the locus.

5. A method as claimed in any one of claims 1 to 4, wherein the locus encodes at least a portion of the variable region of a β-subunit of a T cell receptor.

6. A method as claimed in claim 5, wherein the locus encodes at least a portion of a Vβ₁₂ or Vβ₄ region.

7. A method as claimed in claim 5, wherein the locus is the human equivalent of the mouse locus Vβ_{8.1}, Vβ_{8.2}, Vβ_{8.3} or Vβ₁₇ₐ.

8. A method as claimed in any one of claims 1 to 7, wherein the autoimmune disease comprises the destruction of glial cells and/or myelin.

9. A method as claimed in any one of claims 1 to 7, wherein the autoimmune disease is multiple sclerosis.

10. A method as claimed in any one of claims 1 to 5, wherein the autoimmune disease is rheumatoid arthritis, systemic lupus erythematosus, psoriasis, pemphigus vulgaris, juvenile onset diabetes, Sjögren's disease, thyroid disease, Hashimoto's thyroiditis or myasthenia gravis.

11. A peptide comprising an amino acid sequence encoded by a locus of a T cell receptor variable region associated with the occurrence of an autoimmune disease.

12. A peptide as claimed in claim 11, wherein the amino acid sequence comprises at least 8 amino acids.

13. A peptide as claimed in claim 11 or claim 12, wherein the locus encodes at least a portion of the variable region of a β-subunit of a T cell receptor.

14. A peptide as claimed in claim 13, wherein the locus encodes at least a portion of a Vβ₁₂ or Vβ₄ region.

15. A peptide as claimed in claim 13, wherein the locus is the human equivalent of the mouse locus Vβ_{8.1}, Vβ_{8.2}, Vβ_{8.3} or Vβ₁₇ₐ.

16. A peptide as claimed in any one of claims 11 to 15, wherein the autoimmune disease comprises the destruction of glial cells and/or myelin.

17. A peptide as claimed in claim 16, wherein the autoimmune disease is multiple sclerosis.

18. A peptide as claimed in any one of claims 11 to 13, wherein the autoimmune disease is rheumatoid arthritis, systemic lupus erythematosus, psoriasis, pemphigus vulgaris, juvenile onset diabetes, Sjögren's disease, thyroid disease, Hashimoto's thyroiditis or myasthenia gravis.

19. A method for the production of a pharmaceutical composition for inhibiting lymphocyte mediated attack on human syngeneic cells associated with an autoimmune disease, which comprises admixing a monoclonal antibody specific for an amino acid sequence encoded by a locus of a T cell receptor variable region associated with the autoimmune disease, with a pharmaceutically acceptable carrier.

20. A method as claimed in claim 19, wherein the monoclonal antibody is a humanized antibody.

21. A method as claimed in claim 19 or claim 20, wherein the monoclonal antibody is specific for an amino acid sequence encoded by a locus comprising at least a portion of the variable region of a β-subunit of a T cell receptor.

22. A method as claimed in claim 21, wherein the locus encodes at least a portion of a Vβ₁₂ or Vβ₄ region.

23. A method as claimed in claim 21, wherein the locus is the human equivalent of the mouse locus Vβ_{8.1}, Vβ_{8.2}, Vβ_{8.3} or Vβ₁₇ₐ.

24. A method as claimed in any one of claims 19 to 21, wherein the autoimmune disease comprises the destruction of glial cells and/or myelin.

25. A method as claimed in claim 24, wherein the autoimmune disease is multiple sclerosis.

26. A method as claimed in any one of claims 19 to 25, wherein the autoimmune disease is rheumatoid arthritis, systemic lupus erythematosus, psoriasis, pemphigus vulgaris, juvenile onset diabetes, Sjörgren's disease, thyroid disease, Hashimoto's thyroiditis or myasthenia gravis.

27. A method for the production of a pharmaceutical composition for inhibiting lymphocyte attack on human syngeneic cells associated with an autoimmune disease, which comprises admixing a peptide as claimed in any one of claims 11 to 18, with a pharmaceutically acceptable carrier.

28. A monoclonal antibody specific for an amino acid sequence encoded by a locus of a T cell receptor variable region associated with an autoimmune disease, for use as a medicament.

29. Use of a monoclonal antibody specific for an amino acid sequence encoded by a locus of a T cell receptor variable region associated with an autoimmune disease for the manufacture of a medicament for treatment of an autoimmune disease.

30. Use of a monoclonal antibody as claimed in claim 29, wherein the autoimmune disease comprises the destruction of glial cells and/or myelin.

31. Use as claimed in claim 30, wherein the autoimmune disease is multiple sclerosis.

32. Use of a monoclonal antibody as claimed in claim 29, wherein the autoimmune disease is rheumatoid arthritis, systemic lupus erythematosus, psoriasis, pemphigus vulgaris, juvenile onset diabetes, Sjögren's disease, thyroid disease, Hashimoto's thyroiditis or myasthenia gravis.

33. A peptide as claimed in any one of claims 11 to 18, for use as a medicament.

34. Use of a peptide as claimed in any one of claims 11 to 18, for the manufacture of a medicament for treatment of an autoimmune disease.

35. Use as claimed in claim 34, wherein the autoimmune disease comprises the destruction of glial cells and/or myelin.

36. Use of a peptide as claimed in claim 35, wherein the autoimmune disease is multiple sclerosis.

37. Use as claimed in claim 34, wherein the autoimmune disease is rheumatoid arthritis, systemic lupus erythematosus, psoriasis, pemphigus vulgaris, juvenile onset diabetes, Sjögren's disease, thyroid disease, Hashimoto's thyroiditis or myasthenia gravis.

38. An anti-idiotype antibody to a peptide as claimed in any one of claims 11 to 18.

## Patentansprüche (Patentansprüche für folgende(n) Vertragsstaat(en): AT, BE, CH, DE, FR, GB, IT, LI, LU, NL, SE)

1. Verfahren zum Nachweis der Anfälligkeit eines Menschens für eine Autoimmunerkrankung, bei dem man die Gegenwart eines Locus im Genom eines Menschen nachweist, der für mindestens einen Teil der variablen Region eines T-Zell-Rezeptors kodiert, die mit dem Auftreten der Autoimmunerkrankung assoziiert ist.

2. Verfahren nach Anspruch 1, bei dem man Nukleinsäuren nachweist, die für den Teil der variablen Region des T-Zell-Rezeptors kodieren.

3. Verfahren nach Anspruch 2, bei dem die Nukleinsäure genomische DNA ist.

4. Verfahren nach Anspruch 1, bei dem man das Expressionsprodukt des Locus nachweist.

5. Verfahren nach jeglichem der Ansprüche 1 bis 4, bei dem der Locus mindestens für einen Teil der variablen Region einer β-Untereinheit des T-Zell-Rezeptors kodiert.

6. Verfahren nach Anspruch 5, bei dem der Locus mindestens für einen Teil einer Vβ₁₂- oder Vβ₄-Region kodiert.

7. Verfahren nach Anspruch 5, bei dem der Locus das humane Äquivalent zu dem Locus Vβ_{8.1}, Vβ_{8.2}, Vβ_{8.3} oder Vβ₁₇ₐ der Maus ist.

8. Verfahren nach jeglichem der Ansprüche 1 bis 7, bei dem die Autoimmunerkrankung die Zerstörung von Gliazellen und/oder von Myelin umfaßt.

9. Verfahren nach jeglichem der Ansprüche 1 bis 7, bei dem die Autoimmunerkrankung Mutiple Sklerose ist.

10. Verfahren nach jeglichem der Ansprüche 1 bis 5, bei dem die Autoimmunerkrankung rheumatische Arthritis, systemischer Lupus erythematosus, Psoriasis, Pemphigus vulgaris, juveniler Diabetis, Sjögren-Syndrom, Thyreoiditis, Hashimoto's-Thyreoiditis oder Myasthenia gravis ist.

11. Peptid, welches eine Aminosäure-Sequenz enthält, die von einem Locus für eine variable Region eines T-Zell-Rezeptors kodiert wird, die mit dem Auftreten einer Autoimmunerkrankung assoziiert ist.

12. Peptid nach Anspruch 11, in dem die Aminosäure-Sequenz mindestens 8 Aminosäuren umfaßt.

13. Peptid nach den Ansprüchen 11 oder 12, bei dem der Locus mindestens für einen Teil der variablen Region einer β-Untereinheit eines T-Zell-Rezeptors kodiert.

14. Peptid nach Anspruch 13, bei dem der Locus mindestens für einen Teil einer Vβ₁₂- oder Vβ₄-Region kodiert.

15. Peptid nach Anspruch 13, bei dem der Locus das humane Äquivalent zu dem Locus Vβ_{8.1}, Vβ_{8.2}, Vβ_{8.3} oder Vβ₁₇ₐ der Maus ist.

16. Peptid nach jeglichem der Ansprüche 11 bis 15, bei dem die Autoimmunerkrankung die Zerstörung von Gliazellen und/oder von Myelin umfaßt.

17. Peptid nach Anspruch 16, bei dem die Autoimmunerkrankung Mutiple Sklerosis ist.

18. Peptid nach jeglichem der Ansprüche 11 bis 13, bei dem die Autoimmunerkrankung rheumatische Arthritis, systemischer Lupus erythematosus, Psoriasis, Pemphigus vulgaris, juveniler Diabetis, Sjögren-Syndrom, Thyreoiditis, Hashimoto's-Thyreoiditis oder Myasthenia gravis ist.

19. Pharmazeutische Zusammensetzung zur Hemmung von durch Lymphozyten gesteuerten Angriffen auf humane syngene Zellen, die mit einer Autoimmunerkrankung assoziiert sind, enthaltend einen monoklonalen Antikörper mit Spezifität für eine Aminosäure-sequenz, die von einem Locus für eine variablen Region eines T-Zell-Rezeptors kodiert wird, der mit der Autoimmunerkrankung assoziiert ist, vermischt mit einem pharmazeutisch akzeptablen Träger.

20. Pharmazeutische Zusammensetzung nach Anspruch 19, bei dem der monoklonale Antikörper ein humanisierter Antikörper ist.

21. Pharmazeutische Zusammensetzung nach den Ansprüchen 19 oder 20, bei welcher der monoklonale Antikörper Spezifität für eine Aminosäure-Sequenz besitzt, die von einem Locus für mindestens einen Teil der variablen Region einer β-Untereinheit eines T-Zell-Rezeptors kodiert wird.

22. Pharmazeutische Zusammensetzung nach Anspruch 21, bei der der Locus für mindestens einen Teil des Vβ₁₂- oder Vβ₄-Region kodiert.

23. Pharmazeutische Zusammensetzung nach Anspruch 21, bei der der Locus das humane Äquivalent des Locus Vβ_{8.1}, Vβ_{8.2}, Vβ_{8.3} oder Vβ₁₇ₐ der Maus ist.

24. Pharmazeutische Zusammensetzung nach jeglichem der Ansprüche 19 bis 23, bei der die Autoimmunerkrankung die Zerstörung von Gliazellen und/oder von Myelin umfaßt.

25. Pharmazeutische Zusammensetzung nach Anspruch 24, bei der die Autoimmunerkrankung Mutiple Sklerosis ist.

26. Pharmazeutische Zusammensetzung nach jeglichem der Ansprüche 19 bis 21, bei der die Autoimmunerkrankung rheumatische Arthritis, systemischer Lupus erythematosus, Psoriasis, Pemphigus vulgaris, juveniler Diabetis, Sjögren-Syndrom, Thyreoiditis, Hashimoto's-Thyreoiditis oder Myasthenia gravis ist.

27. Pharmazeutische Zusammensetzung zur Hemmung von durch Lymphozyten gesteuerten Angriffen auf humane syngene Zellen, die mit einer Autoimmunerkrankung assoziiert sind, enthaltend ein Peptid nach jeglichem der Ansprüche 11 bis 18 vermischt mit einem pharmazeutisch akzeptablem Träger.

28. Monoklonaler Antikörper mit Spezifität für eine Aminosäure-Sequenz, die von einem Locus für eine variable Region eines T-Zell-Rezeptors kodiert wird, die mit einer Autoimmunerkrankung assoziiert ist, zur Verwendung als Arzneimittel.

29. Verwendung eines monoklonalen Antikörpers mit Spezifität für eine Aminosäure-Sequenz, die von einem Locus für eine variable Region eines T-Zell-Rezeptors kodiert wird, die mit einer Autoimmunerkrankung assoziiert ist, zur Herstellung eines Arzneimittels zur Behandlung einer Autoimmunerkrankung.

30. Verwendung eines monoklonalen Antikörpers nach Anspruch 29, bei der die Autoimmunerkrankung die Zerstörung von Gliazellen und/oder von Myelin umfaßt.

31. Verwendung nach Anspruch 30, bei der die Autoimmunerkrankung Mutiple Sklerosis ist.

32. Verwendung eines monoklonalen Antikörpers nach Anspruch 29, bei der die Autoimmunerkrankung rheumatische Arthritis, systemischer Lupus erythematosus, Psoriasis, Pemphigus vulgaris, juveniler Diabetis, Sjögren-Syndrom, Thyreoiditis, Hashimoto's-Thyreoiditis oder Myasthenia gravis ist.

33. Peptid nach jeglichem der Ansprüche 11 bis 18, zur Verwendung als Arzneimittel.

34. Verwendung eines Peptids nach jeglichem der Ansprüche 11 bis 18 zur Herstellung eines Arzneimittels zur Behandlung einer Autoimmunerkrankung.

35. Verwendung nach Anspruch 34, bei der die Autoimmunerkrankung die Zerstörung von Gliazellen und/oder von Myelin umfaßt.

36. Verwendung eines Peptids nach Anspruch 35, bei der die Autoimmunerkrankung Mutiple Sklerosis ist.

37. Verwendung nach Anspruch 34, bei der die Autoimmunerkrankung rheumatische Arthritis, systemischer Lupus erythematosus, Psoriasis, Pemphigus vulgaris, juveniler Diabetis, Sjögren-Syndrom, Thyreoiditis, Hashimoto's-Thyreoiditis oder Myasthenia gravis ist.

38. Anti-idiotypischer Antikörper gegen ein Peptid nach jeglichem der Ansprüche 11 bis 18.

## Patentansprüche (Patentansprüche für folgende(n) Vertragsstaat(en): ES)

1. Verfahren zum Nachweis der Anfälligkeit eines Menschens für eine Autoimmunerkrankung, bei dem man die Gegenwart eines Locus im Genom eines Menschen nachweist, der für mindestens einen Teil der variablen Region eines T-Zell-Rezeptors kodiert, die mit dem Auftreten der Autoimmunerkrankung assoziiert ist.

2. Verfahren nach Anspruch 1, bei dem man Nukleinsäuren nachweist, die für den Teil der variablen Region des T-Zell-Rezeptors kodieren.

3. Verfahren nach Anspruch 2, bei dem die Nukleinsäure genomische DNA ist.

4. Verfahren nach Anspruch 1, bei dem man das Expressionsprodukt des Locus nachweist.

5. Verfahren nach jeglichem der Ansprüche 1 bis 4, bei dem der Locus mindestens für einen Teil der variablen Region einer β-Untereinheit des T-Zell-Rezeptors kodiert.

6. Verfahren nach Anspruch 5, bei dem der Locus mindestens für einen Teil einer Vβ₁₂- oder Vβ₄-Region kodiert.

7. Verfahren nach Anspruch 5, bei dem der Locus das humane Äquivalent zu dem Locus Vβ_{8.1}, Vβ_{8.2}, Vβ_{8.3} oder Vβ₁₇ₐ der Maus ist.

8. Verfahren nach jeglichem der Ansprüche 1 bis 7, bei dem die Autoimmunerkrankung die Zerstörung Gliazellen und/oder von Myelin umfaßt.

9. Verfahren nach jeglichem der Ansprüche 1 bis 7, bei dem die Autoimmunerkrankung Mutiple Sklerose ist.

10. Verfahren nach jeglichem der Ansprüche 1 bis 5, bei dem die Autoimmunerkrankung rheumatische Arthritis, systemischer Lupus erythematosus, Psoriasis, Pemphigus vulgaris, juveniler Diabetis, Sjögren-Syndrom, Thyreoiditis, Hashimoto's-Thyreoiditis oder Myasthenia gravis ist.

11. Verfahren zur Herstellung eines Peptids, welches eine Aminosäure-Sequenz enthält, die von einem Locus für eine variable Region eines T-Zell-Rezeptors kodiert wird, die mit dem Auftreten einer Autoimmunerkrankung assoziiert ist, bei dem man das Peptid chemisch synthetisiert oder Wirtszellen, die mit einem Vektor transformiert wurden, der eine DNA-Sequenz, die für das Peptid kodiert, sowie Bereiche zur Initiation und Termination von Translation und Transkription enthält, unter Bedingungen inkubiert, die die Expression der kodierenden Sequenz ermöglichen.

12. Verfahren nach Anspruch 11, bei dem die Aminosäure-Sequenz des Peptids mindestens 8 Aminosäuren umfaßt.

13. Verfahren nach den Ansprüchen 11 oder 12, bei dem der Locus mindestens für einen Teil der variablen Region einer β-Untereinheit eines T-Zell-Rezeptors kodiert.

14. Verfahren nach Anspruch 13, bei dem der Locus mindestens für einen Teil einer Vβ₁₂- oder Vβ₄ -Region kodiert.

15. Verfahren nach Anspruch 13, bei dem der Locus das humane Äquivalent zu dem Locus Vβ_{8.1}, Vβ_{8.2} Vβ_{8.3} oder Vß₁₇ₐ der Maus ist.

16. Verfahren nach jeglichem der Ansprüche 11 bis 13, bei dem die Autoimmunerkrankung die Zerstörung von Gliazellen und/-oder von Myelin umfaßt.

17. Verfahren nach Anspruch 16, bei dem die Autoimmunerkrankung Mutiple Sklerosis ist.

18. Verfahren nach jeglichem der Ansprüche 11 bis 13, bei die Autoimmunerkrankung rheumatische Arthritis, systemischer Lupus erythematosus, Psoriasis, Pemphigus vulgaris, juveniler Diabetis, Sjögren-syndrom, Thyreoiditis, Hashimoto's-Thyreoiditis oder Myasthenia gravis ist.

19. Verfahren zur Herstellung einer pharmazeutischen Zusammensetzung zur Hemmung von durch Lymphozyten gesteuerten Angriffen auf humane syngene Zellen, die mit einer Autoimmunerkrankung assoziiert sind, bei dem man einen monoklonalen Antikörper mit Spezifität für eine Aminosäure-Sequenz, die von einem Locus für eine variable Region eines T-Zell-Rezeptors kodiert wird, die mit der Autoimmunerkrankung assoziiert ist, mit einem pharmazeutisch akzeptablen Träger vermischt.

20. Verfahren nach Anspruch 19, bei dem der monoklonale Antikörper ein humanisierter Antikörper ist.

21. Verfahren nach den Ansprüchen 19 oder 20, bei dem der monoklonale Antikörper Spezifität für eine Aminosäure-Sequenz besitzt, die von einem Locus für mindestens einen Teil der variablen Region einer β-Untereinheit eines T-Zell-Rezeptors kodiert wird.

22. Verfahren nach Anspruch 21, bei dem der Locus für mindestens einen Teil des Vβ₁₂- oder Vβ₄-Region kodiert.

23. Verfahren nach Anspruch 21, bei dem der Locus das humane Äquivalent des Locus Vβ_{8.1}, Vβ_{8.2}, Vβ_{8.3} oder Vβ₁₇ₐ der Maus ist.

24. Verfahren nach jeglichem der Ansprüche 19 bis 21, bei dem die Autoimmunerkrankung die Zerstörung von Gliazellen und/-oder von Myelin umfaßt.

25. Verfahren nach Anspruch 24, bei dem die Autoimmunerkrankung Mutiple Sklerosis ist.

26. Verfahren nach jeglichem der Ansprüche 19 bis 25, bei dem die Autoimmunerkrankung rheumatische Arthritis, systemischer Lupus erythematosus, Psoriasis, Pemphigus vulgaris, juveniler Diabetis, Sjögren-Syndrom, Thyreoiditis, Hashimoto's-Thyreoiditis oder Myasthenia gravis ist.

27. Verfahren zur Herstellung einer pharmazeutischen Zusammensetzung zur Hemmung von durch Lymphozyten gesteuerten Angriffen auf humane syngene Zellen, die mit einer Autoimmunerkrankung assoziiert sind, bei dem man ein Peptid, das nach einem Verfahren der Ansprüche 11 bis 20 hergestellt wurde, mit einem pharmazeutisch akzeptablem Träger vermischt.

28. Verfahren zur Herstellung eines Anti-idiotypischen Antikörpers gegen ein Peptid nach jeglichem der Ansprüche 11 bis 18, bei dem man einen monoklonalen Antikörper gegen den Idiotyp des Antikörpers mit Spezifität für das Peptid erzeugt.

## Patentansprüche (Patentansprüche für folgende(n) Vertragsstaat(en): GR)

1. Verfahren zum Nachweis der Anfälligkeit eines Menschens für eine Autoimmunerkrankung, bei dem man die Gegenwart eines Locus im Genom eines Menschen nachweist, der für mindestens einen Teil der variablen Region eines T-Zell-Rezeptors kodiert, die mit dem Auftreten der Autoimmunerkrankung assoziiert ist.

2. Verfahren nach Anspruch 1, bei dem man Nukleinsäuren nachweist, die für den Teil der variablen Region des T-Zell-Rezeptors kodieren.

3. Verfahren nach Anspruch 2, bei dem die Nukleinsäure aus genomische DNA ist.

4. Verfahren nach Anspruch 1, bei dem man das Expressionsprodukt des Locus nachweist.

5. Verfahren nach jeglichem der Ansprüche 1 bis 4, bei dem der Locus mindestens für einen Teil der variablen Region einer β-Untereinheit des T-Zell-Rezeptors kodiert.

6. Verfahren nach Anspruch 5, bei dem der Locus mindestens für einen Teil einer Vβ₁₂- oder Vβ₄-Region kodiert.

7. Verfahren nach Anspruch 5, bei dem der Locus das humane Äquivalent zu dem Locus Vβ_{8.1}, Vβ_{8.2}, Vβ_{8.3} oder Vβ₁₇ₐ der Maus ist.

8. Verfahren nach jeglichem der Ansprüche 1 bis 7, bei dem die Autoimmunerkrankung die Zerstörung Gliazellen und/oder von Myelin umfaßt.

9. Verfahren nach jeglichem der Ansprüche 1 bis 7, bei dem die Autoimmunerkrankung Mutiple Sklerose ist.

10. Verfahren nach jeglichem der Ansprüche 1 bis 5, bei dem die Autoimmunerkrankung rheumatische Arthritis, systemischer Lupus erythematosus, Psoriasis, Pemphigus vulgaris, juveniler Diabetis, Sjögren-Syndrom, Thyreoiditis, Hashimoto's-Thyreoiditis oder Myasthenia gravis ist.

11. Peptid, welches eine Aminosäure-Sequenz enthält, die von einem Locus für eine variable Region eines T-Zell-Rezeptors kodiert wird, die mit dem Auftreten einer Autoimmunerkrankung assoziiert ist.

12. Peptid nach Anspruch 11, in dem die Aminosäure-Sequenz mindestens 8 Aminosäuren umfaßt.

13. Peptid nach jeglichem der Ansprüche 11 oder 12, bei dem der Locus mindestens für einen Teil der variablen Region einer β-Untereinheit eines T-Zell-Rezeptors kodiert.

14. Peptid nach Anspruch 13, bei dem der Locus mindestens für einen Teil einer Vβ₁₂- oder Vβ₄ -Region kodiert.

15. Peptid nach Anspruch 13, bei dem der Locus das humane Äquivalent zu dem Locus Vβ_{8.1}, Vβ_{8.2} Vβ_{8.3} oder Vβ₁₇ₐ der Maus ist.

16. Peptid nach jeglichem der Ansprüche 11 bis 15, bei dem die Autoimmunerkrankung die Zerstörung von Gliazellen und/oder von Myelin umfaßt.

17. Peptid nach Anspruch 16, bei dem die Autoimmunerkrankung Mutiple Sklerosis ist.

18. Peptid nach jeglichem der Ansprüche 11 bis 13, bei die Autoimmunerkrankung rheumatische Arthritis, systemischer Lupus erythematosus, Psoriasis, Pemphigus vulgaris, juveniler Diabetis, sjögren-syndrom, Thyreoiditis, Hashimoto's-Thyreoiditis oder Myasthenia gravis ist.

19. Verfahren zur Herstellung einer pharmazeutischen Zusammensetzung zur Hemmung von durch Lymphozyten gesteuerten Angriffen auf humane syngene Zellen, die mit einer Autoimmunerkrankung assoziiert sind, bei denen man einen monoklonalen Antikörper mit Spezifität für eine Aminosäure-Sequenz, die von einem Locus für eine variable Region eines T-Zell-Rezeptors kodiert wird, die mit der Autoimmunerkrankung assoziiert ist, mit einem pharmazeutisch akzeptablen Träger vermischt.

20. Verfahren nach Anspruch 19, bei dem der monoklonale Antikörper ein humanisierter Antikörper ist.

21. Verfahren nach den Ansprüchen 19 oder 20, bei dem der monoklonale Antikörper Spezifität für eine Aminosäure-Sequenz besitzt, die von einem Locus für mindestens einen Teil der variablen Region einer β-Untereinheit eines T-Zell-Rezeptors kodiert wird.

22. Verfahren nach Anspruch 21, bei dem der Locus für mindestens einen Teil des Vβ₁₂- oder Vβ₄-Region kodiert.

23. Verfahren nach Anspruch 21, bei dem der Locus das humane Äquivalent des Locus Vβ_{8.1}, Vβ_{8.2}, Vβ_{8.3} oder Vβ₁₇ₐ der Maus ist.

24. Verfahren nach jeglichem der Ansprüche 19 bis 21, bei dem die Autoimmunerkrankung die Zerstörung von Gliazellen und/-oder von Myelin umfaßt.

25. Verfahren nach Anspruch 24, bei dem die Autoimmunerkrankung Mutiple Sklerosis ist.

26. Verfahren nach jeglichem der Ansprüche 19 bis 25, bei dem die Autoimmunerkrankung rheumatische Arthritis, systemischer Lupus erythematosus, Psoriasis, Pemphigus vulgaris, juveniler Diabetis, Sjögren-Syndrom, Thyreoiditis, Hashimoto's-Thyreoiditis oder Myasthenia gravis ist.

27. Verfahren zur Herstellung einer pharmazeutischen Zusammensetzung zur Hemmung von durch Lymphozyten gesteuerten Angriffen auf humane syngene Zellen, die mit einer Autoimmunerkrankung assoziiert sind, bei dem man ein Peptid nach jeglichem der Ansprüche 11 bis 18 mit einem pharmazeutisch akzeptablem Träger vermischt.

28. Monoklonaler Antikörper mit Spezifität für eine Aminosäure-Sequenz, die von einem Locus für eine variablen Region eines T-Zell-Rezeptors kodiert wird und mit einer Autoimmunerkrankung assoziiert ist, zur Verwendung als Arzneimittel.

29. Verwendung eines monoklonalen Antikörpers mit Spezifität für eine Aminosäure-Sequenz, die von einem Locus für eine variable Region eines T-Zell-Rezeptors kodiert wird, die mit einer Autoimmunerkrankung assoziiert ist, zur Herstellung eines Arzneimittels zur Behandlung einer Autoimmunerkrankung.

30. Verwendung eines monoklonalen Antikörpers nach Anspruch 29, wobei die Autoimmunerkrankung die Zerstörung von Gliazellen und/oder von Myelin umfaßt.

31. Verwendung nach Anspruch 30, wobei die Autoimmunerkrankung Mutiple Sklerosis ist.

32. Verwendung eines monoklonalen Antikörpers nach Anspruch 29, bei dem die Autoimmunerkrankung rheumatische Arthritis, systemischer Lupus erythematosus, Psoriasis, Pemphigus vulgaris, juveniler Diabetis, Sjögren-Syndrom, Thyreoiditis, Hashimoto's-Thyreoiditis oder Myasthenia gravis ist.

33. Peptid nach jeglichem der Ansprüche 11 bis 18, zur Verwendung als Arzneimittel.

34. Verwendung eines Peptids nach jeglichem der Ansprüche 11 bis 18 zur Herstellung eines Arzneimittels zur Behandlung einer Autoimmunerkrankung.

35. Verwendung nach 34, wobei die Autoimmunerkrankung die Zerstörung von Gliazellen und/oder von Myelin umfaßt.

36. Verwendung eines Peptids nach Anspruch 35, wobei die Autoimmunerkrankung Mutiple Sklerosis ist.

37. Verwendung nach Anspruch 34, wobei die Autoimmunerkrankung rheumatische Arthritis, systemischer Lupus erythematosus, Psoriasis, Pemphigus vulgaris, juveniler Diabetis, Sjögren-Syndrom, Thyreoiditis, Hashimoto's-Thyreoiditis oder Myasthenia gravis ist.

38. Anti-idiotypischer Antikörper gegen ein Peptid nach jeglichem der Ansprüche 11 bis 18.

## Revendications (Revendications pour l'(les) Etat(s) contractant(s) suivant(s): AT, BE, CH, DE, FR, GB, IT, LI, LU, NL, SE)

1. Une méthode pour détecter la prédisposition d'un hôte humain à une maladie auto-immune, ladite méthode consistant à détecter la présence dans un génome humain d'un locus codant pour au moins une partie de la région variable d'un récepteur de lymphocyte T associé à l'apparition de la maladie auto-immune.

2. Une méthode telle que revendiquée dans la revendication 1, qui consiste à détecter un acide nucléique codant pour la portion de la région variable du récepteur de lymphocyte T.

3. Une méthode telle que revendiquée dans la revendication 2, dans laquelle l'acide nucléique est l'ADN génomique.

4. Une méthode telle que revendiquée dans la revendication 1, qui consiste à détecter le produit d'expression du locus.

5. Une méthode telle que revendiquée dans l'une quelconque des revendications 1 à 4, dans laquelle le locus code pour au moins une portion de la région variable d'une sous-unité β d'un récepteur de lymphocyte T.

6. Une méthode telle que revendiquée dans la revendication 5, dans laquelle le locus code pour au moins une portion d'une région Vβ₁₂ ou Vβ₄.

7. Une méthode telle que revendiquée dans la revendication 5, dans laquelle le locus est l'équivalent humain du locus de souris Vβ_{8.1}, Vβ_{8.2}, Vβ_{8.3} ou Vβ₁₇ₐ.

8. Une méthode telle que revendiquée dans l'une quelconque des revendications 1 à 7, dans laquelle la maladie auto-immune implique la destruction de cellules gliales et/ou de myéline.

9. Une méthode telle que revendiquée dans l'une quelconque des revendications 1 à 7, dans laquelle la maladie auto-immune est la sclérose en plaques.

10. Une méthode telle que revendiquée dans l'une quelconque des revendications 1 à 5, dans laquelle la maladie auto-immune est la polyarthrite rhumatoïde, le lupus érythémateux disséminé, le psoriasis, le pemphigus vulgaire, le diabète juvénile, le syndrome de Sjögren, une affection thyroïdienne, la thyroïdite de Hashimoto ou la myasthénie grave.

11. Un peptide comprenant une séquence d'acides aminés codée par un locus d'une région variable de récepteur de lymphocyte T associée à l'apparition d'une maladie auto-immune.

12. Un peptide tel que revendiqué dans la revendication 11, dans lequel la séquence d'acides aminés comprend au moins 8 acides aminés.

13. Un peptide tel que revendiqué dans la revendication 11 ou la revendication 12, dans lequel le locus code pour au moins une portion de la région variable d'une sous-unité β d'un récepteur de lymphocyte T.

14. Un peptide tel que revendiqué dans la revendication 13, dans lequel le locus code pour au moins une portion d'une région Vβ₁₂ ou Vβ₄.

15. Un peptide tel que revendiqué dans la revendication 13, dans lequel le locus est l'équivalent humain du locus de souris Vβ_{8.1}, Vβ_{8.2}, Vβ_{8.3} ou Vβ₁₇ₐ.

16. Un peptide tel que revendiqué dans l'une quelconque des revendications 11 à 15, dans lequel la maladie auto-immune implique la destruction de cellules gliales et/ou de myéline.

17. Un peptide tel que revendiqué dans la revendication 16, dans lequel la maladie auto-immune est la sclérose en plaques.

18. Un peptide tel que revendiqué dans l'une quelconque des revendications 11 à 13, dans lequel la maladie auto-immune est la polyarthrite rhumatoïde, le lupus érythémateux disséminé, le psoriasis, le pemphigus vulgaire, le diabète juvénile, le syndrome de Sjögren, une affection thyroïdienne, la thyroïdite de Hashimoto ou la myasthénie grave.

19. Une composition pharmaceutique destinée à inhiber une attaque à médiation lymphocytaire de cellules syngéniques humaines associée à une maladie auto-immune, comprenant un anticorps monoclonal spécifique envers une séquence d'acides aminés codée par un locus d'une région variable de récepteur de lymphocyte T associée à la maladie auto-immune, en mélange avec un support pharmaceutiquement acceptable.

20. Une composition pharmaceutique telle que revendiquée dans la revendication 19, dans laquelle l'anticorps monoclonal est un anticorps humanisé.

21. Une composition pharmaceutique selon la revendication 19 ou la revendication 20, dans laquelle l'anticorps monoclonal est spécifique envers une séquence d'acides aminés codée par un locus comprenant au moins une portion de la région variable d'une sous-unité β d'un récepteur de lymphocyte T.

22. Une composition pharmaceutique telle que revendiquée dans la revendication 21, dans laquelle le locus code pour au moins une portion d'une région Vβ₁₂ ou Vβ₄.

23. Une composition pharmaceutique telle que revendiquée dans la revendication 21, dans laquelle le locus est l'équivalent humain du locus de souris Vβ_{8.1}, Vβ_{8.2}, Vβ_{8.3} ou Vβ₁₇ₐ.

24. Une composition pharmaceutique telle que revendiquée dans l'une quelconque des revendications 19 à 23, dans laquelle la maladie auto-immune implique la destruction de cellules gliales et/ou de myéline.

25. Une composition pharmaceutique telle que revendiquée dans la revendication 24, dans laquelle la maladie auto-immune est la sclérose en plaques.

26. Une composition pharmaceutique telle que revendiquée dans l'une quelconque des revendications 19 à 21, dans laquelle la maladie auto-immune est la polyarthrite rhumatoïde, le lupus érythémateux disséminé, le psoriasis, le pemphigus vulgaire, le diabète juvénile, le syndrome de Sjögren, une affection thyroïdienne, la thyroïdite de Hashimoto ou la myasthénie grave.

27. Une composition pharmaceutique destinée à inhiber une attaque lymphocytaire de cellules syngéniques humaines associée à une maladie auto-immune, comprenant un peptide tel que revendiqué dans l'une quelconque des revendications 11 à 18, en mélange avec un support pharmaceutiquement acceptable.

28. Un anticorps monoclonal spécifique envers une séquence d'acides aminés codée par un locus d'une région variable de récepteur de lymphocyte T associée à une maladie auto-immune, pour son utilisation comme médicament.

29. Utilisation d'un anticorps monoclonal spécifique envers une séquence d'acides aminés codée par un locus d'une région variable de récepteur de lymphocyte T associée à une maladie auto-immune pour la fabrication d'un médicament destiné au traitement d'une maladie auto-immune.

30. Utilisation d'un anticorps monoclonal telle que revendiquée dans la revendication 29, dans laquelle la maladie auto-immune implique la destruction de cellules gliales et/ou de myéline.

31. Utilisation telle que revendiquée dans la revendication 30, dans laquelle la maladie auto-immune est la sclérose en plaques.

32. Utilisation d'un anticorps monoclonal telle que revendiquée dans la revendication 29, dans laquelle la maladie auto-immune est la polyarthrite rhumatoïde, le lupus érythémateux disséminé, le psoriasis, le pemphigus vulgaire, le diabète juvénile, le syndrome de Sjögren, une affection thyroïdienne, la thyroïdite de Hashimoto ou la myasthénie grave.

33. Un peptide tel que revendiqué dans l'une quelconque des revendications 11 à 18, pour son utilisation comme médicament.

34. Utilisation d'un peptide tel que revendiqué dans l'une quelconque des revendications 11 à 18 pour la fabrication d'un médicament destiné au traitement d'une maladie auto-immune.

35. Utilisation telle que revendiquée dans la revendication 34, dans laquelle la maladie auto-immune implique la destruction de cellules gliales et/ou de myéline.

36. Utilisation d'un peptide tel que revendiqué dans la revendication 35, dans laquelle la maladie auto-immune est la sclérose en plaques.

37. Utilisation telle que revendiquée dans la revendication 34, dans laquelle la maladie auto-immune est la polyarthrite rhumatoïde, le lupus érythémateux disséminé, le psoriasis, le pemphigus vulgaire, le diabète juvénile, le syndrome de Sjögren, une affection thyroïdienne, la thyroïdite de Hashimoto ou la myasthénie grave.

38. Un anticorps anti-idiotype dirigé contre un peptide tel que revendiqué dans l'une quelconque des revendications 11 à 18.

## Revendications (Revendications pour l'(les) Etat(s) contractant(s) suivant(s): ES)

1. Une méthode pour détecter la prédisposition d'un hôte humain à une maladie auto-immune, ladite méthode consistant à détecter la présence dans un génome humain d'un locus codant pour au moins une partie de la région variable d'un récepteur de lymphocyte T associé à l'apparition de la maladie auto-immune.

2. Une méthode telle que revendiquée dans la revendication 1, qui consiste à détecter un acide nucléique codant pour la portion de la région variable du récepteur de lymphocyte T.

3. Une méthode telle que revendiquée dans la revendication 2, dans laquelle l'acide nucléique est l'ADN génomique.

4. Une méthode telle que revendiquée dans la revendication 1, qui consiste à détecter le produit d'expression du locus.

5. Une méthode telle que revendiquée dans l'une quelconque des revendications 1 à 4, dans laquelle le locus code pour au moins une portion de la région variable d'une sous-unité β d'un récepteur de lymphocyte T.

6. Une méthode telle que revendiquée dans la revendication 5, dans laquelle le locus code pour au moins une portion d'une région Vβ₁₂ ou Vβ₄.

7. Une méthode telle que revendiquée dans la revendication 5, dans laquelle le locus est l'équivalent humain du locus de souris Vβ_{8.1}, Vβ_{8.2}, Vβ_{8.3} ou Vβ₁₇ₐ.

8. Une méthode telle que revendiquée dans l'une quelconque des revendications 1 à 7, dans laquelle la maladie auto-immune implique la destruction de cellules gliales et/ou de myéline.

9. Une méthode telle que revendiquée dans l'une quelconque des revendications 1 à 7, dans laquelle la maladie auto-immune est la sclérose en plaques.

10. Une méthode telle que revendiquée dans l'une quelconque des revendications 1 à 5, dans laquelle la maladie auto-immune est la polyarthrite rhumatoïde, le lupus érythémateux disséminé, le psoriasis, le pemphigus vulgaire, le diabète juvénile, le syndrome de Sjögren, une affection thyroïdienne, la thyroïdite de Hashimoto ou la myasthénie grave.

11. Un procédé pour la production d'un peptide comprenant une séquence d'acides aminés codée par un locus d'une région variable de récepteur de lymphocyte T associée à l'apparition d'une maladie auto-immune, qui consiste à synthétiser chimiquement le peptide, ou à faire incuber une cellule hôte transformée par un vecteur comprenant une séquence d'ADN codant pour le peptide et comprenant également des régions d'initiation et de terminaison de transcription et de traduction, dans des conditions qui assurent l'expression de la séquence codante.

12. Un procédé tel que revendiqué dans la revendication 11, dans lequel la séquence d'acides aminés du peptide comprend au moins 8 acides aminés.

13. Un procédé tel que revendiqué dans la revendication 11 ou la revendication 12, dans lequel le locus code pour au moins une portion de la région variable d'une sous-unité ) d'un récepteur de lymphocyte T.

14. Un procédé tel que revendiqué dans la revendication 13, dans lequel le locus code pour au moins une portion d'une région Vβ₁₂ ou Vβ₄.

15. Un procédé tel que revendiqué dans la revendication 13, dans lequel le locus est l'équivalent humain du locus de souris Vβ_{8.1}, Vβ_{8.2}, Vβ_{8.3} ou Vβ₁₇ₐ.

16. Un procédé tel que revendiqué dans l'une quelconque des revendications 11 à 13, dans lequel la maladie auto-immune implique la destruction de cellules gliales et/ou de myéline.

17. Un procédé tel que revendiqué dans la revendication 16, dans lequel la maladie auto-immune est la sclérose en plaques.

18. Un procédé tel que revendiqué dans l'une quelconque des revendications 11 à 13, dans lequel la maladie auto-immune est la polyarthrite rhumatoïde, le lupus érythémateux disséminé, le psoriasis, le pemphigus vulgaire, le diabète juvénile, le syndrome de Sjögren, une affection thyroïdienne, la thyroïdite de Hashimoto ou la myasthénie grave.

19. Un procédé pour la production d'une composition pharmaceutique destinée à inhiber une attaque à médiation lymphocytaire de cellules syngéniques humaines associée à une maladie auto-immune, qui consiste à mélanger un anti-corps monoclonal spécifique envers une séquence d'acides aminés codée par un locus d'une région variable de récepteur de lymphocyte T associée à la maladie auto-immune, avec un support pharmaceutiquement acceptable.

20. Un procédé tel que revendiqué dans la revendication 19, dans lequel l'anticorps monoclonal est un anticorps humanisé.

21. Un procédé tel que revendiqué dans la revendication 19 ou la revendication 20, dans lequel l'anticorps monoclonal est spécifique envers une séquence d'acides aminés codée par un locus comprenant au moins une portion de la région variable d'une sous-unité β d'un récepteur de lymphocyte T.

22. Un procédé tel que revendiqué dans la revendication 21, dans lequel le locus code pour au moins une portion d'une région Vβ₁₂ ou Vβ₄.

23. Un procédé tel que revendiqué dans la revendication 21, dans lequel le locus est l'équivalent humain du locus de souris Vβ_{8.1}, Vβ_{8.2}, Vβ_{8.3} ou Vβ₁₇ₐ.

24. Un procédé tel que revendiqué dans l'une quelconque des revendications 19 à 21, dans lequel la maladie auto-immune implique la destruction de cellules gliales et/ou de myéline.

25. Un procédé tel que revendiqué dans la revendication 24, dans lequel la maladie auto-immune est la sclérose en plaques.

26. Un procédé tel que revendiqué dans l'une quelconque des revendications 19 à 25, dans lequel la maladie auto-immune est la polyarthrite rhumatoïde, le lupus érythémateux disséminé, le psoriasis, le pemphigus vulgaire, le diabète juvénile, le syndrome de Sjögren, une affection thyroïdienne, la thyroïdite de Hashimoto ou la myasthénie grave.

27. Un procédé pour la production d'une composition pharmaceutique destinée à inhiber une attaque lymphocytaire de cellules syngéniques humaines associée à une maladie auto-immune, qui consiste à mélanger un peptide produit par un procédé tel que revendiqué dans l'une quelconque des revendications 11 à 20 avec un support pharmaceutiquement acceptable.

28. Un procédé pour la production d'un anticorps anti-idiotype dirigé contre un peptide tel que défini dans l'une quelconque des revendications 11 à 18, qui consiste à préparer un anticorps monoclonal dirigé contre l'idiotype de l'anticorps dirigé contre le peptide.

## Revendications (Revendications pour l'(les) Etat(s) contractant(s) suivant(s): GR)

1. Une méthode pour détecter la prédisposition d'un hôte humain à une maladie auto-immune, ladite méthode consistant à détecter la présence dans un génome humain d'un locus codant pour au moins une partie de la région variable d'un récepteur de lymphocyte T associé à l'apparition de la maladie auto-immune.

2. Une méthode telle que revendiquée dans la revendication 1, qui consiste à détecter un acide nucléique codant pour la portion de la région variable du récepteur de lymphocyte T.

3. Une méthode telle que revendiquée dans la revendication 2, dans laquelle l'acide nucléique est l'ADN génomique.

4. Une méthode telle que revendiquée dans la revendication 1, qui consiste à détecter le produit d'expression du locus.

5. Une méthode telle que revendiquée dans l'une quelconque des revendications 1 à 4, dans laquelle le locus code pour au moins une portion de la région variable d'une sous-unité β d'un récepteur de lymphocyte T.

6. Une méthode telle que revendiquée dans la revendication 5, dans laquelle le locus code pour au moins une portion d'une région Vβ₁₂ ou Vβ₄.

7. Une méthode telle que revendiquée dans la revendication 5, dans laquelle le locus est l'équivalent humain du locus de souris Vβ_{8.1}, Vβ_{8.2}, Vβ_{8.3} ou Vβ₁₇ₐ.

8. Une méthode telle que revendiquée dans l'une quelconque des revendications 1 à 7, dans laquelle la maladie auto-immune implique la destruction de cellules gliales et/ou de myéline.

9. Une méthode telle que revendiquée dans l'une quelconque des revendications 1 à 7, dans laquelle la maladie auto-immune est la sclérose en plaques.

10. Une méthode telle que revendiquée dans l'une quelconque des revendications 1 à 5, dans laquelle la maladie auto-immune est la polyarthrite rhumatoïde, le lupus érythémateux disséminé, le psoriasis, le pemphigus vulgaire, le diabète juvénile, le syndrome de Sjögren, une affection thyroïdienne, la thyroïdite de Hashimoto ou la myasthénie grave.

11. Un peptide comprenant une séquence d'acides aminés codée par un locus d'une région variable de récepteur de lymphocyte T associée à l'apparition d'une maladie auto-immune.

12. Un peptide tel que revendiqué dans la revendication 11, dans lequel la séquence d'acides aminés comprend au moins 8 acides aminés.

13. Un peptide tel que revendiqué dans la revendication 11 ou la revendication 12, dans lequel le locus code pour au moins une portion de la région variable d'une sous-unité ) d'un récepteur de lymphocyte T.

14. Un peptide tel que revendiqué dans la revendication 13, dans lequel le locus code pour au moins une portion d'une région Vβ₁₂ ou Vβ₄.

15. Un peptide tel que revendiqué dans la revendication 13, dans lequel le locus est l'équivalent humain du locus de souris Vβ_{8.1}, Vβ_{8.2}, Vβ_{8.3} ou Vβ₁₇ₐ.

16. Un peptide tel que revendiqué dans l'une quelconque des revendications 11 à 15, dans lequel la maladie auto-immune implique la destruction de cellules gliales et/ou de myéline.

17. Un peptide tel que revendiqué dans la revendication 16, dans lequel la maladie auto-immune est la sclérose en plaques.

18. Un peptide tel que revendiqué dans l'une quelconque des revendications 11 à 13, dans lequel la maladie auto-immune est la polyarthrite rhumatoïde, le lupus érythémateux disséminé, le psoriasis, le pemphigus vulgaire, le diabète juvénile, le syndrome de Sjögren, une affection thyroïdienne, la thyroïdite de Hashimoto ou la myasthénie grave.

19. Un procédé pour la production d'une composition pharmaceutique destinée à inhiber une attaque à médiation lymphocytaire de cellules syngéniques humaines associée à une maladie auto-immune, qui consiste à mélanger un anticorps monoclonal spécifique envers une séquence d'acides aminés codée par un locus d'une région variable de récepteur de lymphocyte T associée à la maladie auto-immune, avec un support pharmaceutiquement acceptable.

20. Un procédé tel que revendiqué dans la revendication 19, dans lequel l'anticorps monoclonal est un anticorps humanisé.

21. Un procédé tel que revendiqué dans la revendication 19 ou la revendication 20, dans lequel l'anticorps monoclonal est spécifique envers une séquence d'acides aminés codée par un locus comprenant au moins une portion de la région variable d'une sous-unité β d'un récepteur de lymphocyte T.

22. Un procédé tel que revendiqué dans la revendication 21, dans lequel le locus code pour au moins une portion d'une région Vβ₁₂ ou Vβ₄.

23. Un procédé tel que revendiqué dans la revendication 21, dans lequel le locus est l'équivalent humain du locus de souris Vβ_{8.1}, Vβ_{8.2}, Vβ_{8.3} ou Vβ₁₇ₐ.

24. Un procédé tel que revendiqué dans l'une quelconque des revendications 19 à 21, dans lequel la maladie auto-immune implique la destruction de cellules gliales et/ou de myéline.

25. Un procédé tel que revendiqué dans la revendication 24, dans lequel la maladie auto-immune est la sclérose en plaques.

26. Un procédé tel que revendiqué dans l'une quelconque des revendications 19 à 25, dans lequel la maladie auto-immune est la polyarthrite rhumatoïde, le lupus érythémateux disséminé, le psoriasis, le pemphigus vulgaire, le diabète juvénile, le syndrome de Sjögren, une affection thyroïdienne, la thyroïdite de Hashimoto ou la myasthénie grave.

27. Un procédé pour la production d'une composition pharmaceutique destinée à inhiber une attaque lymphocytaire de cellules syngéniques humaines associée à une maladie auto-immune, qui consiste à mélanger un peptide tel que revendiqué dans l'une quelconque des revendications 11 à 18 avec un support pharmaceutiquement acceptable.

28. Un anticorps monoclonal spécifique envers une séquence d'acides aminés codée par un locus d'une région variable de récepteur de lymphocyte T associée à une maladie auto-immune, pour son utilisation comme médicament.

29. Utilisation d'un anticorps monoclonal spécifique envers une séquence d'acides aminés codée par un locus d'une région variable de récepteur de lymphocyte T associée à une maladie auto-immune pour la fabrication d'un médicament destiné au traitement d'une maladie auto-immune.

30. Utilisation d'un anticorps monoclonal telle que revendiquée dans la revendication 29, dans laquelle la maladie auto-immune implique la destruction de cellules gliales et/ou de myéline.

31. Utilisation telle que revendiquée dans la revendication 30, dans laquelle la maladie auto-immune est la sclérose en plaques.

32. Utilisation d'un anticorps monoclonal telle que revendiquée dans la revendication 29, dans laquelle la maladie auto-immune est la polyarthrite rhumatoïde, le lupus érythémateux disséminé, le psoriasis, le pemphigus vulgaire, le diabète juvénile, le syndrome de Sjögren, une affection thyroïdienne, la thyroïdite de Hashimoto ou la myasthénie grave.

33. Un peptide tel que revendiqué dans l'une quelconque des revendications 11 à 18, pour son utilisation comme médicament.

34. Utilisation d'un peptide tel que revendiqué dans l'une quelconque des revendications 11 à 18 pour la fabrication d'un médicament destiné au traitement d'une maladie auto-immune.

35. Utilisation telle que revendiquée dans la revendication 34, dans laquelle la maladie auto-immune implique la destruction de cellules gliales et/ou de myéline.

36. Utilisation d'un peptide tel que revendiqué dans la revendication 35, dans laquelle la maladie auto-immune est la sclérose en plaques.

37. Utilisation telle que revendiquée dans la revendication 34, dans laquelle la maladie auto-immune est la polyarthrite rhumatoïde, le lupus érythémateux disséminé, le psoriasis, le pemphigus vulgaire, le diabète juvénile, le syndrome de Sjögren, une affection thyroïdienne, la thyroïdite de Hashimoto ou la myasthénie grave.

38. Un anticorps anti-idiotype dirigé contre un peptide tel que revendiqué dans l'une quelconque des revendications 11 à 18.
